(19) Europäisches Patentamt
European Patent Office
Office européen des brevets

(11) EP 4 065 592 B1

(12) **EUROPEAN PATENT SPECIFICATION**

(45) Date of publication and mention of the grant of the patent:
**10.01.2024 Bulletin 2024/02**

(21) Application number: **20811327.4**

(22) Date of filing: **24.11.2020**

(51) International Patent Classification (IPC):
***C07J 9/00** (2006.01)* ***C07C 401/00** (2006.01)*
***C07C 39/08** (2006.01)* ***A61K 31/592** (2006.01)*
***A61K 31/593** (2006.01)* ***A61K 31/575** (2006.01)*
***A61P 3/02** (2006.01)* ***A61P 19/10** (2006.01)*
***A61P 35/00** (2006.01)* ***A61P 19/08** (2006.01)*

(52) Cooperative Patent Classification (CPC):
**C07J 9/00; A61P 3/02; A61P 19/08; A61P 19/10; A61P 35/00; C07C 39/08; C07C 401/00;**
C07B 2200/13

(86) International application number:
**PCT/EP2020/083254**

(87) International publication number:
**WO 2021/105147 (03.06.2021 Gazette 2021/22)**

(54) **COCRYSTALS OF STEROID AND SECOSTEROID COMPOUNDS AND COMPOSITIONS COMPRISING THEM**

CO-KRISTALLE VON STEROID- UND SECOSTEROIDVERBINDUNGEN UND ZUSAMMENSETZUNGEN DAMIT

COCRISTAUX DE COMPOSÉS DE STÉROÏDES ET DE SECOSTÉROÏDES ET COMPOSITIONS LES COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.11.2019 EP 19383040**

(43) Date of publication of application:
**05.10.2022 Bulletin 2022/40**

(73) Proprietor: **Center for Intelligent Research in Crystal Engineering, S.L.**
**07121 Palma de Mallorca (ES)**

(72) Inventors:
- **BOFILL HERRERA, Lidia**
**08145 BIGUES I RIELLS (ES)**
- **DE SANDE LÓPEZ, Dafne**
**08904 L'HOSPITALET DE LLOBREGAT (ES)**
- **PROHENS LÓPEZ, Rafel**
**08201 SABADELL (ES)**
- **BARBAS CAÑERO, Rafael**
**08924 SANTA COLOMA DE GRAMENET (ES)**

(74) Representative: **ZBM Patents - Zea, Barlocci & Markvardsen**
**Rambla Catalunya, 123**
**08008 Barcelona (ES)**

(56) References cited:
**WO-A1-2019/154237 WO-A2-2008/153945**

- **BHUPINDER SINGH SEKHON: "Nutraceutical Cocrystals: An overview", RGUHS J PHARM SCI, vol. 2, no. 2, 1 January 2012 (2012-01-01) , pages 16-25, XP055067370, DOI: 10.5530/rjps.2012.2.3**
- **JIAN-RONG WANG ET AL: "Stabilizing vitamin D 3 by conformationally selective co-crystallization", CHEMICAL COMMUNICATIONS, vol. 50, no. 7, 1 January 2014 (2014-01-01), pages 855-858, XP055692073, ISSN: 1359-7345, DOI: 10.1039/C3CC47747A cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 065 592 B1

- **JIAN-RONG WANG ET AL: "Drug-drug co-crystallization presents a new opportunity for the development of stable vitamins", CHEMICAL COMMUNICATIONS, vol. 52, no. 17, 1 January 2016 (2016-01-01), pages 3572-3575, XP055692076, ISSN: 1359-7345, DOI: 10.1039/C5CC10297A cited in the application**

## Description

This application claims the benefit of European Patent Application 19383040.3 filed on November 25, 2019.

## Technical Field

The present invention relates to cocrystals of steroid and secosteroid compounds, to processes for the preparation thereof, and to their use as a medicament or a dietary supplement. It also relates to compositions comprising them.

## Background Art

Vitamin D is a group of fat-soluble secosteroids responsible for increasing intestinal absorption of calcium, and phosphate, and other biological functions. In humans, the most important compounds in this group are vitamin $D_3$ (also known as cholecalciferol) and vitamin $D_2$ (ergocalciferol). Cholecalciferol and ergocalciferol are naturally present in very few foods, added to others, and available as a dietary supplement. The major natural source of the vitamin is synthesis of cholecalciferol in the skin from cholesterol through a chemical reaction that is dependent on sun exposure.

Vitamin D has a significant role in calcium homeostasis and metabolism. Vitamin D supplements are given to treat or to prevent rickets in children and osteomalacia in adults. Together with calcium, vitamin D also helps protect older adults from osteoporosis.

Vitamin $D_3$ is converted in the liver to calcifediol (25-hydroxycholecalciferol); ergocalciferol is converted to ercalcidiol (25-hydroxyergocalciferol). These two vitamin D metabolites (generically called 25-hydroxyvitamin D) are measured in serum to determine a person's vitamin D status. Calcifediol is further hydroxylated by the kidneys to form calcitriol (also known as 1 ,25-dihydroxycholecalciferol), the biologically active form of vitamin D. Calcitriol circulates as a hormone in the blood, having a major role regulating the concentration of calcium and phosphate, and promoting the healthy growth and remodeling of bone. Calcitriol also has other effects, including some on cell growth, neuromuscular and immune functions, and reduction of inflammation. Similarly, Vitamin $D_2$ is finally metabolized via actions of the liver and kidneys to its active form ercalcitriol (also known as 1,25-dihydroxyergocalciferol). Ercalcitriol has been found to exhibit substantially greater antirachitic activity than the vitamin $D_2$. Besides, alfacalcidol (or 1-hydroxycholecalciferol) is an active vitamin $D_3$ metabolite used for supplementation in humans and as a poultry feed additive. It is a more useful form of vitamin D supplementation, mostly due to much longer half-life and lower kidney load, since it does not require the second hydroxylation step in the kidney.

Cholecalciferol, ergocalciferol, calcifediol, ercalcidiol, calcitriol, ercalcitriol, and alfacalcidol have the following chemical structures:

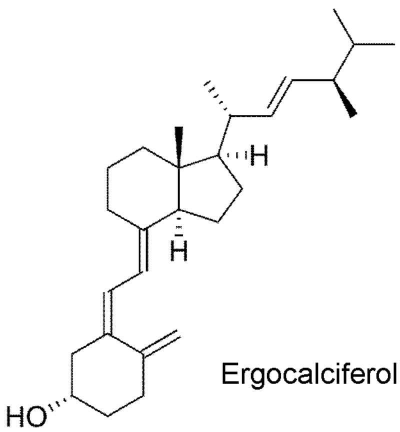

Ergocalciferol

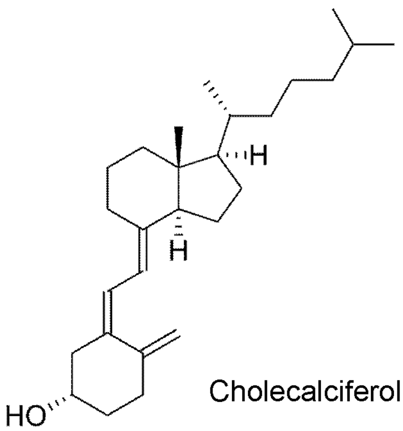

Cholecalciferol

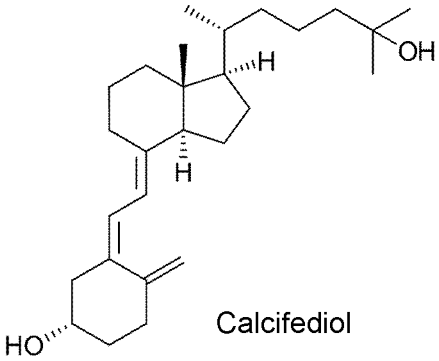

Calcifediol

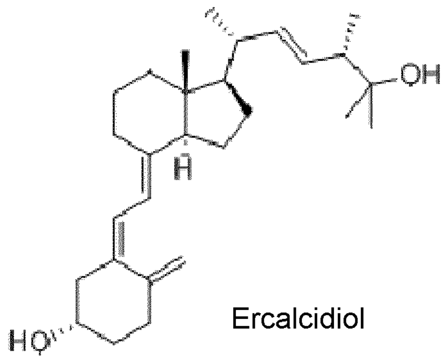

Ercalcidiol

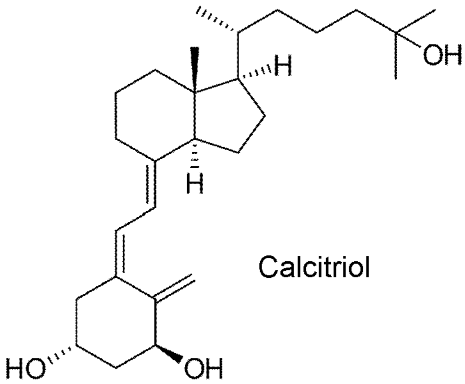

Calcitriol

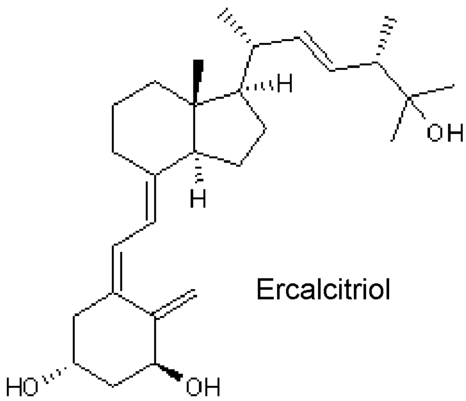

Ercalcitriol

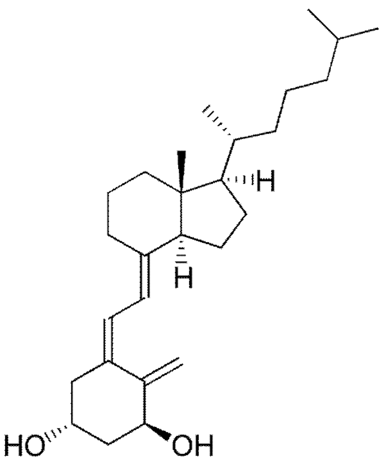

Alfacalcidol

Also of interest are ergosterol and 7-dehydrocholesterol, which are provitamins forms of vitamin $D_2$ and vitamin $D_3$ , respectively, and whose chemical formula is the following:

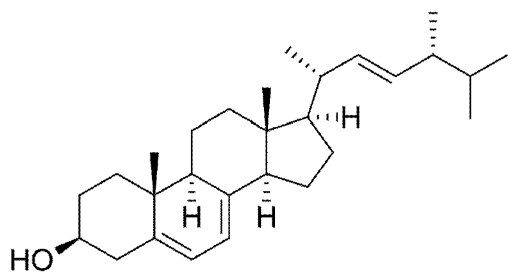

Ergosterol

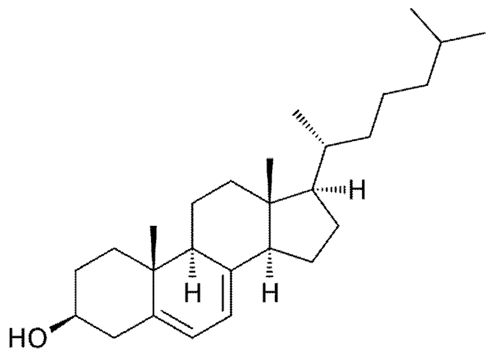

7-Dehidrocholesterol

All these steroid or secosteroid molecules are unstable, being quite sensitive to light and heat and losing their activity by decomposition in air. Thus, they must be stored under controlled conditions for storage and transportation and they are rarely formulated in solid dosage form due to its unstable nature in the solid-state.

Several attempts to stabilize this kind of molecules have been made. As an instance, in Wang, JR *et al.* 2013, certain stabilization of vitamin $D_3$ is achieved by forming a co-crystal with cholesterol or cholestanol. Similarly, the same authors (Wang, JR *et al.* 2016) have obtained a co-crystal of vitamin $D_2$ and vitamin $D_3$ by conformationally selective co-crystallization as a 1:1 heterodimeric complex by combining $\alpha$-vitamin $D_2$ and $\beta$-vitamin $D_3$ to match hydrogen-bond donors with acceptors and to accommodate structural complementarities.

WO-A-2008/153945 discloses a number of nutraceuticals (including Vitamin D2 and D3) and co-crystal formers (including phenol compounds). However, neither Vitamin D2, Vitamin D3 nor phenol co-formers were chosen for inclusion in any specific co-crystals in this document, which also fails to provide any evidence of phenol stabilisation of Vitamin D2 or D3.

WO-A-2019/154237 achieved stabilisation by co-crystallisation of Vitamin D2 with Vitamin D3. No phenols were used as crystal co-formers.

A basic requirement for satisfactory bioavailability is that the active ingredient is able to dissolve adequately in the digestive tract. The compounds mentioned above are usually provided in the pharmaceutical and health food sector in the form of tablets or capsules. Nevertheless, its low solubility still poses some problems regarding to its bioavailability.

It is known that different solid forms of an active ingredient can have different characteristics, and some of them can offer certain advantages, for example regarding stability, solubility, or bioavailability. Thus, the discovery of new solid forms allows improving the pharmacokinetic properties of the active ingredients and, consequently, the characteristics of the pharmaceutical formulations containing the active ingredients, since some forms are more adequate for one type of formulation, and other forms for other different formulations.

Particularly, in recent years cocrystal formation has emerged as a viable strategy towards improving the pharmacokinetic data of active ingredients. By cocrystallizing an active ingredient or a salt of an active ingredient with a coformer (the second component of the cocrystal), a new solid state form of the active ingredient can be created. The new solid form can have unique properties compared with existing solid forms of the active ingredient or its salts. Such different properties may provide a basis for improving formulations, for example, by facilitating better processing or handling characteristics, changing the dissolution profile in a favourable direction, or improving stability and shelf-life. However, cocrystal formation is not predictable, and in fact is not always possible. Moreover, there is no way to predict the properties of a particular cocrystal of a compound until it is formed. Finding the appropriate coformers and right conditions to obtain a particular cocrystal can take significant time, effort and resources.

From what is known in the art, there is still the need of finding new more stable and soluble solid forms of the above mentioned steroid and secosteroid compounds in order to improve the pharmaceutical properties of the pharmaceutical formulations containing them, particularly in terms of stability and bioavailability.

## Summary of Invention

The inventors have found that some steroid and secosteroid compounds can form a cocrystal with a hydrogen bond donor coformer which is orcinol or resorcinol as defined herein below. The provision of the mentioned cocrystals gives a new tool to overcome the problems associated with the water solubility of the corresponding steroid and secosteroid compounds because it has been found that these cocrystals have a better water solubility and higher dissolution rate in aqueous media than the corresponding steroid or secosteroid compounds, what makes them more bioavailable. Additionally, as shown in the examples, the mentioned cocrystals have improved photostability and stability under stress conditions. These properties make the cocrystals more suitable for preparing pharmaceutical or dietary compositions

containing such steroid and secosteroid compounds.

Cocrystal formation, particularly with the hydrogen bond donor coformers cannot be predicted. Accordingly, the provision of improved crystal forms of steroid and secosteroid compounds such as ergocalciferol, cholecalciferol, calcifediol, ercalcidiol, calcitriol, ercalcitriol, alfacalcidol, ergosterol, and 7-dehydrocholesterol in the form of cocrystals with a hydrogen bond donor coformer such as orcinol and resorcinol is considered a contribution to the art.

Thus, a first aspect of the invention refers to the provision of a cocrystal of a compound of formula (I) or of a compound formula (II)

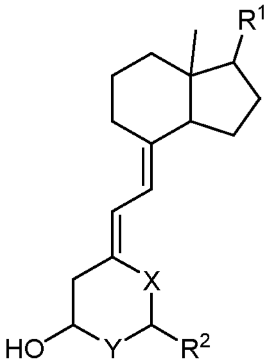
(I)

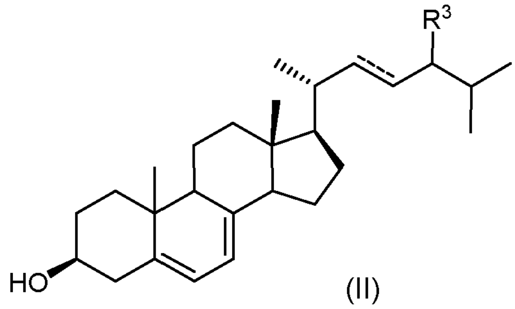
(II)

wherein

X and Y are independently selected from $-CH_2-$ and $-C(CH_2)-$, provided that al least one of X or Y is $-CH_2-$;

$R^1$ is $-CHCH_3-Z_m-W_n-(CH_2)_o-T_p-S$, wherein

Z is O, and m is 0 or 1;

W is $R^4CH$- - -$CHR^5$, wherein either $R^4$ and $R^5$ are H and the dashed line indicates that there is a single bond, or $R^4$ and $R^5$ together are forming a bond and the dashed line indicates that there is a double bond, and n is 0 or 1;

o is 0, 1 or 3;

T is selected from the group consisting of $-CHR^6-$ and -C(O), wherein $R^6$ is -OH or $-CH_3$, and p is 0 or 1;

S is selected from the group consisting of H, $(C_1-C_3)$ alkyl (particularly isopropyl) optionally substituted by -OH, $(C_1-C_3)$ haloalkyl (particularly 1,1,1,3,3,3-hexafluoro-2-propyl) optionally substituted by -OH, cyclopropyl, and

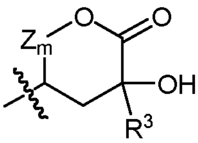 ;

and

$R^2$ is -H or -OH;

$R^3$ is H or $CH_3$; and

the dashed line in formula (II) indicates a single or a double bond;

and a hydrogen bond donor coformer which is a phenolic compound such as orcinol or resorcinol.

A second aspect of the invention refers to a composition comprising an effective amount of the cocrystal of a compound of formula (I) or of formula (II) and a hydrogen bond donor coformer as defined above together with one or

more appropriate acceptable excipients or carriers.

A third aspect of the invention refers to a personal care product product comprising the composition as defined herein above en below.

Finally, a fourth aspect of the invention refers to a cocrystal of a compound of formula (I) or of formula (II) and a hydrogen bond donor coformer as defined herein above and below for use as a medicament.

**Brief Description of Drawings**

Fig. 1 shows the X-ray powder diffractogram (XRPD) of $VD_3$:resorcinol cocrystal polymorph A (1:1). $VD_3$ stands for vitamin $D_3$.

Fig. 2 shows the XRPD of $VD_3$:resorcinol cocrystal polymorph B (1:1).

Fig. 3 shows the XRPD of $VD_2$:resorcinol cocrystal. $VD_2$ stands for vitamin $D_2$.

Fig. 4 shows the XRPD of cocrystal $VD_3$:orcinol.

Fig. 5 shows the XRPD of $VD_2$:orcinol cocrystal (polymorph A).

Fig. 6 shows the XRPD of $VD_2$:orcinol cocrystal (polymorph B).

Fig. 7 shows the XRPD of $VD_2$:beta-sitosterol (2:1).

Fig. 8 shows the XRPD of $VD_2$:propionic acid cocrystal (2:1).

Fig. 9 shows the changes in Vitamin $D_2$ content during storage at 23 °C with an illuminance of 5000 lx.

Fig. 10 shows the changes in Vitamin $D_3$ content during storage at 23 °C with an illuminance of 5000 lx.

Fig. 11 shows the dissolution profiles of $VD_2$, $VD_3$, $VD_2$-$VD_3$ and $VD_3$-resorcinol solids forms.

Fig. 12 shows the remaining $VD_3$ or $VD_3$:resorcinol after 12, 32, 60 and 90 days at 25°C/57% humidity, or after 12 days at 40°C/75% humidity.

XRPD diagrams express intensity (I; counts) versus angle 2 theta (°).

**Detailed description of the invention**

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present invention, ranges given, such as of temperatures, times, and the like, should be considered approximate, unless specifically stated.

For the purposes of the invention, the term "cocrystal" refers herein to a crystalline entity with at least two different components constituting the unit cell at room temperature (20-25 °C) and interacting by intermolecular interactions. Thus, in a cocrystal, one component crystallizes together with one or more neutral components.

The expression "cocrystal obtainable by" is used here to define each specific cocrystal of the invention by the process for obtaining it and refers to the product obtainable by any of the corresponding processes disclosed herein. For the purposes of the invention the expressions "obtainable", "obtained" and equivalent expressions are used interchangeably and, in any case, the expression "obtainable" encompasses the expression "obtained".

The term "hydrogen-bond donor coformer" refers to a compound having hydrogen atoms bound to an electronegative atom (such as nitrogen, oxygen, or sulfur) and with the ability to stablish strong intermolecular hydrogen-bonds with a hydrogen-bond acceptor. The term "hydrogen-bond acceptor" refers to a compound having at least one electronegative atom (such as oxygen, nitrogen, or halogen) capable of interacting with an electropositive hydrogen atom through a hydrogen bond.

The term "alkyl" refers to a saturated straight, or branched hydrocarbon chain which contains the number of carbon atoms specified in the description or claims. Examples include, among others, the group methyl, ethyl, propyl, and isopropyl. Alkyl groups can be unsubstituted or substituted as described herein, being the substituents placed on any available position.

The terms "halo" or "halogen", by themselves or as part of another substituent, mean, unless otherwise stated, a fluorine, chlorine, bromine, or iodine atom. Additionally, terms such as "haloalkyl", are meant to include monohaloalkyl and polyhaloalkyl. For example, the term "$C_1$-$C_3$ haloalkyl" is meant to include trifluoromethyl, 2,2,2-trifluoroethyl, 4-chlorobutyl, 3-bromopropyl, and 1,1,1,3,3,3-hexafluoro-2-propyl, the later being particularly preferred.

When values of characteristic peaks of an X-ray diffractogram are given it is said that these are "approximate" values. It should be understood that the values are the ones shown in the corresponding lists or tables $\pm$ 0.3 degrees 2 theta measured in an X-ray diffractometer with Cu-$K_\alpha$ radiation $\lambda$=1.5418 Å.

When a ratio of components of the cocrystals of the invention is specified it refers to the molar ratio of the components that forms the cocrystal. The term "molar ratio" has been used to express the stoichiometric amount in moles of each of the components of a cocrystal. The molar ratio can be determined by $^1$H NMR (Proton nuclear magnetic resonance), thermogravimetric analysis (TGA) or single crystal X-ray diffraction (SCXRD).

The term "room temperature" refers to a temperature of the environment, without heating or cooling, and is generally from 20 °C to 25 °C.

The term "overnight" refers to a time interval of from 10 h to 20 h.

It is noted that, as used in this specification and the appended claims, the singular forms "a", "an", and "the" include plural referents unless the context clearly dictates otherwise.

As mentioned above, the first aspect of the invention is the provision of cocrystal of a compound of formula (I) or of formula (II) as defined above and a hydrogen bond donor coformer which is a phenolic compound such as orcinol or resorcinol.

In an embodiment of the first aspect of the invention, m is 0. In another embodiment, m, n and p are 0, o is 1, and S is

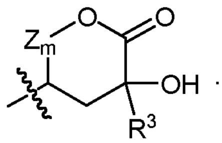

wherein $R^3$ is as defined above.

In another embodiment of the first aspect of the invention, m is 0, n is 1 and S is selected from the group consisting of H, ($C_1$-$C_3$) alkyl (particularly isopropyl) optionally substituted by -OH, ($C_1$-$C_3$) haloalkyl (particularly 1,1,1,3,3,3-hexafluoro-2-propyl) optionally substituted by -OH, and cyclopropyl.

In another embodiment of the first aspect of the invention, $R^1$ is selected from the group consisting of

- $CHCH_3$-$(CH_2)_3$-$CH(CH_3)_2$,
- $CHCH_3$-$(CH_2)_5$-$C(CH_3)_2OH$,
- $CHCH_3$-$(CH_2)_3$-$C(CH_3)_2OH$,
- $CHCH_3$-$(CH_2)_2$-$CH_2OH$-$C(CH_3)_2OH$,
- $CHCH_3$-$(CH_2)_2$-$CH_2OH$-$CH(CH_3)_2$,
- $CHCH_3$-$(CH_2)_2$-CO-$C(CH_3)_2OH$,
- $CHCH_3$-$(CH_2)_2$-CO-$CH(CH_3)_2$,
- $CHCH_3$-CH=CH-$CHCH_3$-$CH(CH_3)_2$,
- $CHCH_3$-CH=CH-$CHCH_3$-$C(CH_3)_2OH$,
- $CHCH_3$-$CH_2$-$CH_3$,

- $CHCH_3-(CH_2)_3-C(CF_3)_2OH$,
- $CHCH_3-O-(CH_2)_2-C(CH_3)_2OH$,
- $CHCH_3-CH=CH-CHOH-(CH)_3$,

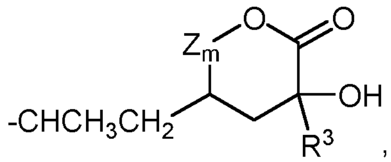

wherein Z, $R^3$ and m are as defined above.

In another embodiment, the compound of formula (I) is selected from the group consisting of ergocalciferol, cholecalciferol, calcifediol, ercalcidiol, calcitriol, ercalcitriol, alfacalcidol, tacalcitol, becocalcidiol, doxercalciferon, paricalcitol, calcipotriene, calcitroic acid, calcitetrol, 24-oxo-1alpha,25-dihydroxyvitamin D3, calcitriol-26,23-lactone, 1a,25-dihydroxy-cholecalciferol-26,23-peroxylactone, 26,26,26,27,27,27-hexafluoro- 1alpha,25-dihydroxycholecalciferol, 24-hydroxyvitamin D3, 1alpha-hydroxy-24-oxocholecalciferol, calcifediol lactone, (23S,25R)-25-hydroxycholecalciferol-26,23-peroxylactone, 1,25-dihydroxy-24-oxo-vitamin D3, 24,25- dihydroxycholecalciferol, 25-hydroxy-24-oxo-cholecalciferol, and maxacalcitol; and the compound of formula (II) is selected from ergosterol and 7-dehidrocholesterol.

In another embodiment, the cocrystal is a cocrystal of a compound of formula (I) selected from the group consisting of ergocalciferol, cholecalciferol, calcifediol, ercalcidiol, calcitriol, ercalcitriol, and alfacalcidol; and a phenolic compound.

In another embodiment, the cocrystal is a cocrystal of ergosterol or 7-dehidrocholesterol and a phenolic compound.

In an embodiment, optionally in combination with one or more features of the particular embodiments defined above, the hydrogen bond donor coformer is a phenolic compound, particularly an orcinol or resorcinol.

In another embodiment, the phenolic compound is resorcinol and the cocrystal is cocrystal $VD_3$ and resorcinol polymorph A which is characterized by having an X-ray powder diffractogram that comprises characteristic peaks at approximately 3.6 and 17.0 $\pm$ 0.3 degrees 2 theta (Cu-$K_\alpha$ radiation, $\lambda$ = 1.5418 Å). In an embodiment, the cocrystal of $VD_3$ and resorcinol polymorph A of the invention is characterized by having an X-ray powder diffractogram that further comprises characteristic peaks at 11.2, 14.6 and 16.2 $\pm$ 0.3 degrees 2 theta (Cu-$K_\alpha$ radiation, $\lambda$ = 1.5418 Å).

More specifically, cocrystal $VD_3$ and resorcinol polymorph A is characterized by exhibiting in the X-ray powder diffractogram a pattern of peaks, expressed in 2 theta units in degrees, 2$\theta$ (°), which is shown in Table 1.

Table 1: List of selected peaks (only peaks with relative intensity greater than or equal to 1% are indicated):

| **Pos.** [°2Th.] | **Rel.** Int. [%] |
|---|---|
| 3.5557 | 86.9 |
| 7.1031 | 1.85 |
| 8.8861 | 12.22 |
| 10.205 | 3.53 |
| 11.2358 | 52.28 |
| 12.4358 | 15.41 |
| 14.2056 | 5.11 |
| 14.6249 | 26.68 |
| 14.9464 | 9.72 |
| 15.4682 | 7.47 |
| 16.1696 | 61.6 |
| 16.6788 | 28.8 |
| 17.0359 | 100 |
| 17.3108 | 31.19 |
| 17.5323 | 30 |
| 17.7721 | 46.34 |
| 18.0268 | 18.28 |

(continued)

| **Pos.** [°2Th.] | **Rel.** Int. [%] |
|---|---|
| 18.3846 | 11.33 |
| 19.1441 | 29.1 |
| 19.591 | 8.63 |
| 21.057 | 8.44 |
| 22.1658 | 5.35 |
| 22.8201 | 21.55 |
| 23.0291 | 6.77 |
| 23.3722 | 18.44 |
| 23.8622 | 5.51 |
| 24.4407 | 6.21 |
| 27.4303 | 7.15 |
| 30.5995 | 9.26 |

Cocrystal $VD_3$ and resorcinol polymorph A may be further characterized by an X-ray diffractogram as in FIG. 1.

In another embodiment, the phenolic compound is resorcinol and the cocrystal is cocrystal of $VD_3$ and resorcinol polymorph B which is characterized by having an X-ray powder diffractogram that comprises characteristic peaks at approximately 3.5 and 17.5 ± 0.3 degrees 2 theta (Cu-$K_\alpha$ radiation, $\lambda$ = 1.5418 Å). In an embodiment, the cocrystal of $VD_3$ and resorcinol polymorph B of the invention is characterized by having an X-ray powder diffractogram that further comprises characteristic peaks at 9.4, 11.2 and 15.7 ± 0.3 degrees 2 theta (Cu-$K_\alpha$ radiation, $\lambda$ = 1.5418 Å).

More specifically, the cocrystal of $VD_3$ and resorcinol polymorph B of the present disclosure is characterized by exhibiting in the X-ray powder diffractogram a pattern of peaks, expressed in 2 theta units in degrees, 2$\theta$ (°), which is shown in Table 2.

Table 2: List of selected peaks (only peaks with relative intensity greater than or equal to 1% are indicated):

| **Pos. [°2Th.]** | **Rel. Int. [%]** |
|---|---|
| 3.5121 | 100 |
| 7.0611 | 2.33 |
| 9.3967 | 26.3 |
| 11.2281 | 54.09 |
| 13.756 | 6.9 |
| 14.1806 | 9.63 |
| 14.4488 | 9.73 |
| 14.6733 | 28.44 |
| 15.7142 | 50.51 |
| 16.6739 | 46.9 |
| 16.9052 | 48.8 |
| 17.0974 | 62.83 |
| 17.4732 | 97.78 |
| 17.8455 | 24.08 |
| 18.5082 | 37.86 |
| 18.8918 | 22.25 |
| 19.5187 | 7.58 |
| 19.816 | 7.36 |
| 20.5003 | 24.09 |
| 20.9378 | 6.58 |
| 22.7477 | 23.44 |
| 23.6647 | 11.52 |
| 24.1567 | 5.58 |
| 24.9865 | 3.77 |
| 26.6257 | 13.09 |

(continued)

| Pos. [°2Th.] | Rel. Int. [%] |
|---|---|
| 28.4853 | 4.84 |
| 29.1555 | 5.34 |
| 30.5169 | 5.98 |
| 32.001 | 3.14 |

Cocrystal $VD_3$ and resorcinol polymorph B may be further characterized by an X-ray diffractogram as in FIG. 2.

In another embodiment, the phenolic compound is resorcinol and the cocrystal is cocrystal of $VD_2$ and resorcinol which is characterized by having an X-ray powder diffractogram that comprises characteristic peaks at approximately 3.4 and 17.0 $\pm$ 0.3 degrees 2 theta (Cu-$K_\alpha$ radiation, $\lambda$ = 1.5418 Å). In another embodiment, the cocrystal of cocrystal of $VD_2$ and resorcinol is characterized by having an X-ray powder diffractogram that further comprises characteristic peaks at 11.1, 15.5, and 16.3 $\pm$ 0.3 degrees 2 theta (Cu-$K_a$ radiation, $\lambda$ = 1.5418 Å).

More specifically, the cocrystal of $VD_2$ and resorcinol of the present disclosure is characterized by exhibiting in the X-ray powder diffractogram a pattern of peaks, expressed in 2 theta units in degrees, 2$\theta$ (°), which is shown in Table 3.

Table 3: List of selected peaks (only peaks with relative intensity greater than or equal to 1% are indicated):

| Pos. [°2Th.] | Rel. Int. [%] |
|---|---|
| 3.4426 | 100 |
| 6.8571 | 4.19 |
| 8.9277 | 7.08 |
| 10.1549 | 6.39 |
| 11.1191 | 30.53 |
| 12.2469 | 8.92 |
| 13.6994 | 7.11 |
| 14.9856 | 23.66 |
| 15.4683 | 45.31 |
| 16.2717 | 42.35 |
| 17.028 | 73.26 |
| 17.3677 | 43.75 |
| 17.6116 | 18.28 |
| 17.8599 | 7.41 |
| 18.9022 | 10.34 |
| 18.9022 | 10.34 |
| 19.5311 | 7.2 |
| 19.8988 | 10.74 |
| 20.3623 | 4.53 |
| 20.8612 | 7.66 |
| 22.4078 | 2.98 |
| 22.8716 | 4.79 |
| 23.1277 | 7.37 |
| 23.4393 | 5.67 |
| 23.897 | 5.78 |
| 24.234 | 8.86 |
| 27.8047 | 2.85 |
| 28.2209 | 3.38 |
| 29.397 | 6.49 |
| 30.7228 | 4.07 |

The cocrystal of $VD_2$ and resorcinol of the present disclosure may be further characterized by an X-ray diffractogram as in FIG. 3.

In another embodiment, the phenolic compound is orcinol and the cocrystal is the cocrystal of $VD_3$ and orcinol

which is characterized by having an X-ray powder diffractogram that comprises characteristic peaks at approximately 3.3 and 17.7 ± 0.3 degrees 2 theta (Cu-$K_a$ radiation, λ = 1.5418 Å). In another embodiment, the cocrystal of $VD_3$ and orcinol of the present disclosure is characterized by having an X-ray powder diffractogram that further comprises characteristic peaks at 9.9, 11.4 and 16.0 ± 0.3 degrees 2 theta (Cu-$K_\alpha$ radiation, λ = 1.5418 Å).

More specifically, the cocrystal of $VD_3$ and orcinol of the present disclosure is characterized by exhibiting in the X-ray powder diffractogram a pattern of peaks, expressed in 2 theta units in degrees, 2θ (°), which is shown in Table 4.

Table 4: List of selected peaks (only peaks with relative intensity greater than or equal to 1% are indicated):

| Pos. [°2Th.] | Rel. Int. [%] |
|---|---|
| 3.2756 | 100 |
| 6.5193 | 2.05 |
| 7.207 | 1.1 |
| 7.6186 | 2.18 |
| 9.8746 | 21.67 |
| 11.3682 | 29.49 |
| 13.5005 | 4.9 |
| 14.4209 | 11.97 |
| 15.2374 | 7.36 |
| 16.0216 | 26.7 |
| 17.2023 | 16.23 |
| 17.7451 | 54.72 |
| 18.1937 | 15.97 |
| 18.6688 | 14.43 |
| 19.7831 | 3.47 |
| 20.4617 | 5.37 |
| 20.726 | 5.6 |
| 21.0948 | 8.71 |
| 22.4295 | 5.69 |
| 22.7491 | 9.36 |
| 23.2177 | 4.33 |
| 24.0603 | 2.75 |
| 24.6764 | 2.41 |
| 24.8689 | 4.13 |
| 25.0754 | 3.15 |
| 25.8751 | 2.53 |
| 28.8817 | 2.45 |
| 29.8323 | 2.46 |
| 31.7361 | 2.29 |

The cocrystal of $VD_3$ and orcinol of the present disclosure may be further characterized by an X-ray diffractogram as in FIG. 4.

In another embodiment, the cocrystal of $VD_2$ and orcinol polymorph A of the present disclosure is characterized by having an X-ray powder diffractogram that comprises characteristic peaks at approximately 3.2 and 17.5 ± 0.3 degrees 2 theta (Cu-$K_a$ radiation, λ = 1.5418 Å). In another embodiment, the cocrystal of $VD_2$ and orcinol polymorph A of the present disclosure is characterized by having an X-ray powder diffractogram that further comprises characteristic peaks at 9.8, 11.2 and 15.7 ± 0.3 degrees 2 theta (Cu-$K_a$ radiation, λ = 1.5418 Å). Particularly, the molar ratio of $VD_2$ and orcinol is 1:1.

More specifically, the cocrystal of $VD_2$ and orcinol polymorph A of the present disclosure is characterized by exhibiting in the X-ray powder diffractogram a pattern of peaks, expressed in 2 theta units in degrees, 2θ (°), which is shown in Table 5.

Table 5: List of selected peaks (only peaks with relative intensity greater than or equal to 1% are indicated):

| Pos. [°2Th.] | Rel. Int. [%] |
|---|---|
| 3.1632 | 100 |
| 5.2574 | 1.82 |
| 6.3217 | 5.53 |
| 9.8018 | 41.83 |
| 11.2318 | 41.99 |
| 12.66 | 3.21 |
| 13.2807 | 6.46 |
| 14.4638 | 10.33 |
| 14.8114 | 27.21 |
| 15.7114 | 45.23 |
| 17.207 | 60.6 |
| 17.509 | 98.82 |
| 18.3585 | 11.78 |
| 18.6069 | 23.75 |
| 19.28 | 15.38 |
| 19.6904 | 9.92 |
| 20.9205 | 9.92 |
| 21.3985 | 13.95 |
| 22.2391 | 1.59 |
| 22.5599 | 7.16 |
| 23.4526 | 12.97 |
| 23.9518 | 9.14 |
| 24.5035 | 10.54 |
| 25.4993 | 2.05 |
| 25.7339 | 3.37 |
| 26.6215 | 2.34 |
| 26.8976 | 3.26 |
| 28.2378 | 2.66 |
| 29.1439 | 7.87 |
| 31.856 | 4.6 |

The cocrystal of $VD_2$ and orcinol polymorph A of the present disclosure may be further characterized by an X-ray diffractogram as in FIG. 5.

The data of the structure of the cocrystal $VD_2$ and orcinol polymorph A defined above obtained by single crystal X-ray diffraction are shown below:

| **Structure** | **$VD_2$ and orcinol polymorph A** |
|---|---|
| Temperature (K) | 304(2) |
| Wavelength (Å) | 0.71073 |
| Crystal system | Monoclinic |
| Space group | P 2 1 |
| a, b, c (Å) | 18.3814(13), 9.5459(6), 28.663(2) |
| $\alpha$, $\beta$, $\gamma$ (°) | 90, 102.513(3), 90 |
| Volume ($Å^3$) | 4910.0(6) |
| Z, Density (calc.) ($Mg/m^3$) | 6, 1.057 |
| Final R indices [$I > 2\sigma(I)$] | R1 = 0.0973, wR2 = 0.1693 |

In another embodiment, the cocrystal of $VD_2$ and orcinol polymorph B of the present present disclosure is characterized by having an X-ray powder diffractogram that comprises characteristic peaks at approximately 3.1 and 17.3 ± 0.3 degrees 2 theta (Cu-$K_\alpha$ radiation, λ = 1.5418 Å). In another embodiment, the cocrystal of $VD_2$ and orcinol polymorph B of the present disclosure is characterized by having an X-ray powder diffractogram that further comprises characteristic peaks at 9.7, 14.6 and 17.0 ± 0.3 degrees 2 theta (Cu-$K_\alpha$ radiation, λ = 1.5418 Å). Particularly, the molar ratio of $VD_2$ and orcinol is 1:1.

More specifically, the cocrystal of $VD_2$ and orcinol polymorph B of the present disclosure is characterized by exhibiting in the X-ray powder diffractogram a pattern of peaks, expressed in 2 theta units in degrees, 2θ (°), which is shown in Table 6.

Table 6: List of selected peaks (only peaks with relative intensity greater than or equal to 1% are indicated):

| Pos. [°2Th.] | Rel. Int. [%] |
|---|---|
| 3.1494 | 100 |
| 6.3291 | 5.3 |
| 7.2614 | 0.82 |
| 8.6481 | 1.86 |
| 9.741 | 23.81 |
| 10.6399 | 0.32 |
| 11.1929 | 24.37 |
| 11.7503 | 0.53 |
| 12.2478 | 0.92 |
| 12.7075 | 3.8 |
| 13.2647 | 5.18 |
| 14.2234 | 5.75 |
| 14.5779 | 18.73 |
| 15.7269 | 35.79 |
| 16.9783 | 35.58 |
| 17.2823 | 58.16 |
| 18.1443 | 7.29 |
| 18.501 | 13.35 |
| 19.1298 | 14.15 |
| 19.5111 | 8.42 |
| 20.8513 | 8.31 |
| 21.2671 | 13.64 |
| 22.5198 | 6.4 |
| 22.7905 | 6.42 |
| 23.3578 | 14.17 |
| 23.9146 | 9.47 |
| 24.2888 | 5.96 |
| 25.6839 | 3.68 |
| 26.5732 | 3.39 |
| 26.7428 | 4.44 |
| 28.6805 | 5.03 |
| 29.3903 | 3.53 |

The cocrystal of $VD_2$ and orcinol polymorph B of the present disclosure may be further characterized by an X-ray diffractogram as in FIG. 6.

It is also disclosed a cocrystal of $VD_2$ and beta-sitosterol which is characterized by having an X-ray powder diffractogram that comprises characteristic peaks at approximately 2.5 and 15.6 ± 0.3 degrees 2 theta (Cu-$K_a$ radiation, λ = 1.5418 Å). Particularly, the cocrystal of $VD_2$ and beta-sitosterol is characterized by having an X-ray powder diffractogram that further comprises characteristic peaks at 5.0, 8.7, 12.5 and 14.7 ± 0.3 degrees 2 theta (Cu-$K_a$ radiation, λ= 1.5418 Å).

More specifically, the cocrystal of $VD_2$ and beta-sitosterol of the present disclosure is characterized by exhibiting

in the X-ray powder diffractogram a pattern of peaks, expressed in 2 theta units in degrees, 2θ (°), which is shown in Table 7.

Table 7: List of selected peaks (only peaks with relative intensity greater than or equal to 1% are indicated):

| Pos. [°2Th.] | Rel. Int. [%] |
|---|---|
| 2.4587 | 100 |
| 3.5631 | 15.8 |
| 4.0233 | 5.09 |
| 4.9518 | 33.35 |
| 6.0364 | 7.92 |
| 8.7215 | 18.79 |
| 12.019 | 17.8 |
| 12.4654 | 26.52 |
| 12.655 | 9.7 |
| 13.4025 | 8.99 |
| 13.9072 | 26.89 |
| 14.6596 | 60.29 |
| 14.9134 | 12 |
| 15.0804 | 9.64 |
| 15.3827 | 7.54 |
| 15.6257 | 72.58 |
| 15.8282 | 43.26 |
| 16.4068 | 49.28 |
| 17.5333 | 75.7 |
| 18.0097 | 33.5 |
| 18.2834 | 5.26 |
| 19.987 | 7.74 |
| 20.1733 | 10.84 |
| 20.4972 | 7.49 |
| 22.3944 | 6.64 |
| 22.6588 | 8.95 |
| 25.506 | 5.4 |
| 26.215 | 1.69 |
| 28.3857 | 1.92 |

The cocrystal of $VD_2$ and beta-sitosterol of the present disclosure may be further characterized by an X-ray diffractogram as in FIG. 7.

It is also part of the present disclosure the provision of a process for the preparation of the cocrystal of a compound of formula (I) or of a compound formula (II) as defined above and a hydrogen bond donor coformer which is a phenolic compound such as orcinol or resorcinol, said process comprising the steps of:

a) either preparing a solution of the compound of formula (I) or of a compound formula (II) in an organic solvent selected from the group consisting of methanol, ethanol, isopropanol, butanol, methyl ethyl ketone, methyl isobutyl ketone, acetone, acetonitrile, dimethylformamide, pentane, heptane, cyclohexane, toluene, xylene, ethyl acetate, diethyl ether, tetrahydrofuran, ethylene glycol dimethyl ether, diisopropyl ether, dioxane, dichloromethane, chloroform, acetic acid, benzyl alcohol, formic acid, dimethyl sulfoxide, ethylene glycol, water, aqueous ammonia, diethylamine, and mixtures thereof; and adding the phenolic compound until a suspension is observed;

or, alternatively, preparing a concentrated solution of the phenolic compound in the organic solvent and adding the compound of formula (I) or of a compound formula (II) until a suspension is observed;

or, alternatively, preparing a suspension of the compound of formula (I) or of a compound formula (II) and the phenolic compound in the organic solvent;

b) stirring the suspension at room temperature until the cocrystal is formed; and

c) isolating the cocrystal thus obtained;
or

a') preparing a suspension of the compound of formula (I) or of a compound formula (II) and the phenolic compound in an organic solvent selected from the group consisting of methanol, ethanol, isopropanol, butanol, methyl ethyl ketone, methyl isobutyl ketone, acetone, acetonitrile, dimethylformamide, pentane, heptane, cyclohexane, toluene, xylene, ethyl acetate, diethyl ether, tetrahydrofuran, ethylene glycol dimethyl ether, diisopropyl ether, dioxane, dichloromethane, chloroform, acetic acid, benzyl alcohol, formic acid, dimethyl sulfoxide, ethylene glycol, water, aqueous ammonia, diethylamine, and mixtures thereof until a suspension is observed; adding another solvent until total dissolution; and removing the solvent under vacuum until a crystallized solid is obtained;
or

a") grinding the compound of formula (I) or of a compound formula (II) and the phenolic compound together in order to obtain the cocrystal.

Particularly, in step a) the solvent is selected from methyl ethyl ketone, acetonitrile and toluene, and in step a') the organic solvent is pentane.

The isolation step c) may include removing the organic solvent, for example, by one or more of the following operations: filtration, filtration under vacuum, decantation, and centrifugation, or other suitable techniques as known to a person skilled in the art. Particularly, step c) is carried out by filtration of the solid. In another embodiment, step c) further comprises drying the isolated cocrystal. More particularly, the cocrystal is dried at room temperature, more particularly under vacuum conditions. Generally, the vacuum involves a pressure from 0.5 mbar to 3 mbar.

The cocrystals of a compound of formula (I) or of a compound formula (II) as defined above and a hydrogen bond donor coformer which is a phenolic compound such as orcinol or resorcinol as defined above may also be defined by its preparation process. Accordingly, this aspect of the invention can be formulated as the cocrystals of a compound of formula (I) or of a compound formula (II) as defined above and a hydrogen bond donor coformer which is a phenolic compound such as orcinol or resorcinol by the process disclosed above and, specifically, by the particular processes for each of the cocrystals disclosed in the examples.

Formulations for the stabilization of the cocrystal are also object of the present invention. Vitamin cocrystals can be formulated using conventional formulation techniques including, without being limited to, silica adsorbing, ethylcellulose coating, fat coating, drum drying, spray drying, spray congealing, and cross-linked spray congealing, in order to obtain, for example, pulverized particles, granulates, microspheres, flakes, pulverized round particles, spherical or spheroidal beadlets, flakes, irregular or spherical agglomerates, encapsulated oil.

Depending on the technique and final result the usual additives for each technique can be used. The same additive may play different roles depending on the final formulation. For example, gelatin can be used as emulsifier, as texturing agent, or as encapsultating agent.

Examples of additives that can be used in vitamin formulations include, without being limited to, tricalciumphosphate, silicon dioxide, starches (modified or not), proteins from animal sources (including gelatins), proteins from plant sources, casein, pectin, alginate, agar, guar, maltodextrins, lignin sulfonates, cellulose derivatives, sugars, saccharides, sorbitols, gums, and mixtures thereof.

For oil containing formulations, examples of oils include, without being limited to, babassu, coconut, cohune, sunflower, murumyru tallow, palm kernel, tucum oil, and mixtures thereof.

Small quantities of other ingredients may be incorporated including, without being limited to, antioxidants such as 6-ethoxy-1,2-dihydroxy-2,2,4-trimethylquinoline (ethoxyquine), 3,5-di-tertiary-4-butyl hydroxytoluene (BHT), 3-tertiary butyl-hydroxyanisole (BHA), tocopherols, humectants such as glycerol, sorbitol, polyethylene glycol, and propylene glycol, extenders and solubilizers.

As mentioned above, a second aspect of the invention refers to a composition comprising an effective amount of the cocrystal of a compound of formula (I) or of formula (II) and a hydrogen bond donor coformer as defined above together with one or more appropriate acceptable excipients or carriers. The term "effective amount" refers to the amount of the cocrystal which provides a therapeutic effect after its application.

Particularly, the composition is a pharmaceutical or veterinary composition, a cosmetical composition, a dietary supplement including functional food and functional beverages, a medical food, a premix, an animal feed, or a pet food. Additionally, the composition of the present disclosure can be included in a personal care product other than the listed above.

In an embodiment, the composition of the second aspect of the invention is a pharmaceutical composition

comprising a pharmaceutically effective amount of the cocrystal of a compound of formula (I) or of formula (II) and a hydrogen bond donor coformer as defined above together with one or more appropriate pharmaceutically acceptable excipients or carriers. The pharmaceutical composition facilitates administration of the cocrystal to an organism. Particularly, the pharmaceutical composition can be formulated for inhaled, intramuscular, subcutaneous, oral, or topical, administration. Pharmaceutical compositions encompass also veterinary compositions.

In another embodiment, the composition of the second aspect of the invention is a dietary supplement comprising an effective amount of the cocrystal of a compound of formula (I) or of formula (II) and a hydrogen bond donor coformer as defined above together with one or more appropriate orally acceptable excipients or carriers. The term "dietary supplement" refers to a product taken orally that contains an ingredient intended to supplement the diet, including an animal feed supplement. Dietary supplements can be in form of tablets, capsules, softgels, gelcaps, liquids, powders, bars, drinks, shakes and other food products. As an example, the dietary supplement may be to enhance athletic performance.

The term "pharmaceutically acceptable excipients or carriers" or "dietary acceptable excipients or carriers" refers to pharmaceutically or dietary acceptable materials, compositions or vehicles, respectively. Each component must be pharmaceutically or dietary acceptable in the sense of being compatible with the other ingredients of the pharmaceutical or dietary composition, respectively. It must also be suitable for use in contact with the tissue or organ of humans and animals without excessive toxicity, irritation, allergic response, immunogenicity or other problems or complications commensurate with a reasonable benefit/risk ratio.

In an embodiment, the composition of the second aspect of the invention is a cosmetical composition comprising a safe and effective amount of the cocrystal of a compound of formula (I) or of formula (II) and a hydrogen bond donor coformer as defined above together with one or more cosmetically acceptable excipients or carriers.

The term "safe and effective amount" is defined as any amount sufficient to significantly improving the cosmetic appearance of the skin without substantial irritation, but low enough to avoid serious side effects (at a reasonable benefit/risk ratio), within the scope of sound medical judgement. The safe and effective amount of [active ingredients] will vary with the age and physical condition of the consumer, the condition of the skin, the duration of the treatment, the nature of any concurrent treatment, the specific combination of active ingredients employed, the particular cosmetically acceptable carrier utilized, and like factors in the knowledge and expertise of any attending physician.

The term "cosmetically acceptable" refers to that excipients or carriers suitable for use in contact with human skin without undue toxicity, incompatibility, instability, allergic response, among others.

In an embodiment, the composition of the second aspect of the invention is a premix, particularly, a premix comprising minerals, choline chloride or a combination thereof. Minerals are known to accelerate vitamins degradation. Choline chloride is highly aggressive towards other nutrients, including vitamins.

The term "premix" refers to a composition comprising microingredients such as vitamins, minerals, nucleotides, amino acids, chemical preservatives, antibiotics, fermentation products, and other ingredients such as carotenoids and polyphenols. Premixes are for blending into commercial rations, for fortification of food products, or for the preparation of animal feeds.

In an embodiment, the composition of the second aspect of the invention is a medical food. Medical foods are foods that are specially formulated and intended for the dietary management of a disease that has distinctive nutritional needs that cannot be met by normal diet alone.

In a particular embodiment, the pharmaceutical composition as defined above further comprises one or more active ingredients selected from the group consisting of antiosteoporotic agents, antipsoriatic agents, chemotherapeutic agents, immunomodulatory agents, cancer hormone therapeutic agents, targeted cancer therapeutic agents, antidiabetic agents, antihypertensive agents, multiple sclerosis therapeutic agents, antiviral agents, anti-Alzheimer agents, anti-Parkinson agents, therapeutic agents for topical dermatitis, and agents for the treatment of macular degeneration. In another particular embodiment, the dietary supplement as defined above further comprises one or more active ingredients selected from the group consisting of L-carnitine, xylitol, vitamins, carotenoids, flavonoids, copper, zinc, and manganese.

Examples of antiosteoporotic agents include bisphosphonates, such as alendronate, risedronate, ibandronate, etidronate, zoledronate or zolendronic acid; teriparatide, abaloparatide and parathyroid hormone, raloxifene, calcitonin, denosumab, strontium ranelate, and hormone replacement therapy, such as with estrogen.

Examples of antipsoriatic agents include topical corticosteroids, such as clobetasol propionate, betamethasone dipropionate, halobetasol propionate, diflorasone diacetate, fluocinonide, halcinonide, amcinonide, desoximetasone, triamcinolone acetonide, mometsone furoate, fluticasone propionate, halometasone, fluocinolone acetonide, hydrocortisone valerate, hydrocortisone butyrate, flurandrenolide, desonide, alclometasone dipropionate, hydrocortisone; vitamin D analogues , such as calcifediol, calcijex, calcitriol, doxercalciferol, hectorol, paricalcitol, rayaldee, rolcatrol, zemplar; anthralin; topical retinoids, such as retinol, tretinoin, adapalene, tazarotene, alitretinoin, bexarotene; calcineurin inhibitors, such as tacrolimus, pimecrolimus; Janus Kinase (JAK) inhibitors, such as ruxolitinib, tofacitinib, oclacitinib, baricitinib, peficitinib, fedratinib, upadacitinib, filgotinib, cerdulatinib, gandotinib, lestaurtinib, momelotinib, pacritinib, PF-04965842, curcubitazin, CHZ868; salicylic acid, coal tar and moisturizers.

Examples of chemotherapeutic agents include alkylating agents, such asmechlorethamine, cyclophosphamide, melphalan, chlorambucil, ifosfamide, busulfan, N-nitroso-N-mdthylurea, carmustine, lomustine, semustine, fotemustine, streptozotocin, dacarbazide, mitozolomide, temozolomide, thiotepa, mitomycin, diaziquone, cisplatin, carboplatin, oxaliplatin, procarbazine, hexamethylmelamine ;antimetabolites, such as methotrexate, pemetrexed, fluorouracil, capecitabine, cytarabine, gemcitabine, decitabine, azacytidine, fludarabine, nelarabine, cladribine, clofarabine, pentostatin, thioguanine, mercaptopurine;anti-microtubule agents, such as vincristine, vinblastine, vinorelbine, vindesine, vinflunine, paclitaxel, docetaxel, podophyllotoxin, etoposide, teniposide; topoisomerase inhibitors, such as irinotecan, topotecan, etoposide, doxorubicin, mitoxantrone, teniposide, novobiocin, merbarone, aclarubicin; cytotoxic antibiotics, such as mitomycin C, actinomycin, doxorubicin, daunorubicin, epirubicin, aclarubicin, mitoxantrone, bleomycin; and janus kinase inhibitors such as previously described.

Examples of immunomodulatory agents include ipilimumab, nivolumab, pembrolizumab, atezolizumab, avelumab, durvalumab, cemiplimab, rituximab, oncorine, talimogene, laherparecvec, tisagenlecleucel, axicabtagene, ciloleucel, interferon alpha 2a, interferon alpha 2b, human leukocyte interferon-alpha, interferon beta 1a, interferon beta, 1b, interferon PEGylated versions, interleukin-2, interleukin-7, interleukin-12, chemokine ligand -3, chemokine ligand-26, chemokine ligand-7, BCG vaccine, thalidomide, lenalidomide, pomalidomide, apremilast, cytosine phosphate-guanosine, oligodeoxynucleotides, and glucans,sipuleucel-T vaccine.

Examples of cancer hormone therapeutic agents include tamoxifen, anastrozole, letrozole, exemestane, raloxifene, and fulvestrant.

Examples of targeted cancer therapeutic agents include imatinib, gefitinib, erlotinib, sorafenib, sunitinib, dasatinib, lapatinib, nilotinib, bortezomib, tamoxifen, tofacitinib, crizotinib, obatoclax, navitoclax, gossypol, iniparib, olaparib, perifosine, apatinib, vemurafenib, dabrafenib, trametinib, salinomycin, vintafolide, temsitolimus, everolimus, vemurafenib, trametinib, dabrafenib, prembolizumab, rituximab, trastuzumab, alemtuzumab, cetuximab, panitumumab, bevacizumab, and ipilimumab.

Examples of antidiabetic agents include biguanides (i.e., metformin, buformin, phenformin), sulfonylureas (i.e., acetohexamide, carbutamide, chlorpropamide, glibomuride, gliclazide, glimepiride, glipizide, gliquidone, glisoxepid, glyburide, glybuthiazole, glybuzole, glyhexamide, glymidine, glypinamide, phenbutamide, tolazamide, tolbutamide, tolcyclomide), thiazolidinediones (i.e., piogliatazone, rosiglitazone, troglitazone), beta andrenergic blockers, and other antidiabetics such as acarbose, calcium mesoxalate, miglitol, nateglinide, repaglinide, voglibose.

Examples of antihypertensive agents include diuretics, such as bumetanide, ethacrynic acid, furosemide, torsemide, epitizide, hydrochlorothiazide, chlorothiazide, Bendroflumethiazide, methyclothiazide, polythiazide, indapamide, chlorthadolide, metalozone, xipmide, clopamide, amiloride, triamterene, spironolactone, eplerenone; calcium channel blockers such as amplodipine, cilnidipine, clevidipine, felodipine, isradipine, lercanidipine, nicardipine, nifedipine, nimodipine, nisoldipine, nitrendipine, diltiazem, verapamil; angiotensin-converting enzyme inhibitors such as captopril, enalapril, fosinopril, lisinopril, moexiproil, perindopril, quinapril, ramipril, trandolapril, benazepril; angiotensin II receptor antagonists such as azilsartan, candesartan, eprosartan, ibersartan, losartan, Olmesartan, telmisartan, valsartan, fimasartan; adrenergic receptor antagonists such as acebutonol, atenolol, bisoprolol, betaxodol, carteolol, carvediol, labetalol, metoprolol, nadolol, nebivolol, oxprenolol, penbutolol, pindolol, propranolol, timolol, doxazosin, phentolamine, indoramin, phenoxybenzamine, prazosin, terazosin, tolazoline, bucindolol, carvedilol, labetalol; vasodilators such as sodium nitroprusside, hydralazine, dihydralazine, cadralazine, endralazine; renin inhibitors, such as aliskiren; aldosterose receptor antagonists such as eplerenone, spironolactone; alpha-2 adrenergic receptor agonists such as clonidine, guanabenz, guanfacine, methyldopa, moxonidine, guanethidine, mecamylamine, reserpine, kinoxidil; and endothelium receptor blockers, such as bosentan.

Examples of multiple sclerosis therapeutic agents include corticosteroids such as prednisolone, methylprednisolone; modifying progression treatments, such as ocrelizumab, natalizumab, alemtuzumab, mitoxantrone, beta interferons, glatiramer acetate, fingolimod, dimethyl fumarate, teriflunomide, siponimod; signs and simptoms treatments such as muscle relaxants (i.e. baclofen, tizanidine); fatigue redactors (i.e. amantadine, modafinil, methylphenidate; and agents increasing walking speed (i.e. dalfampridine).

Examples of antiviral agents include abacavir, acyclovir, adefovir, amantadine, ampligen, amprenavir, arbidol, atazanavir, atripla, balavir, baloxavir marboxil, bikarvy, cidofovir, combivir, darunavir, delavirdine, descovy, didanosine, docosanol, dolutegravir, ecoliever, edoxudine, efavirenz, emtricitabine, enfuvirtide, entecavir, famciclovir, fomivirsen, fosamprenavir, foscarnet, fosfonet, ibacitabine, idoxuridine, imiquimod, imunovir, indinavir, inosine, integrase inhibitor, interferon (type I, II, III), lamivudine, lopinavir, loviride, nelfinavir, nevirapine, nexavir, nitazoxanide, norvir, nucleoside analogues, oseltamivir, peginterferon alfa-2a, penciclovir, peramivir, pleconaril, podophyllotoxin, protease inhibitor, pyramidine, raltegravir, reverse transcriptase inhibitor, ribavirin, rimantadine, ritonavir, saquinavir, sofosbuvir, stavudine, telaprevir, tenofovir alafenamide, tenofovir disoproxil, tenofovir, tipranavir, trifluridine, trizivir, tromantadine, truvada, valaciclovir, valganciclovir, vicriviroc, vidarabine, viramidine, zalcitabine, zanamivir, and zidovudine.

Examples of anti-Alzheimer agents include cholinesterase inhibitors such as donepezil, galantamine, rivastigmine, tacrine, physostigmine, neostigmine, pyridostigmine, ambenonium, demacarium, caffeine, rosmarinic acid, alpha-pipene, edrophonium, huperize A, ladostigil, ungeremine, lactucopicrin, acotiamide; and NMDA receptor antagonists such as memantine.

Examples of anti-Parkinson agents include carbidopa-levodopa; dopamine agonists, such as pramipexole, ropinirole, rotigotine, apomorphine, lergotrile, pergolide, bromocriptine, lisuride, aripiprazole, phencyclidine, quinpirole, salvinorin A, cabergolide, ciladopa, dihydrexidine, dinapsoline, doxanthrine, epicriptine, piribedil, pramipexole, propylnorapomorphine, quinagolide, roxindole, sumanirole, fenoldopam; MAO B inhibitors such as isocarboxacid, nialamine, phenelzine, hydracarbazine, tranylcypromine, bifemelane, moclobemide, pirlindole, toloxatone, rasagiline, selegiline, safinamide, linezolidbenmoxin, iproclozide, iproniazid, menabazine, octamonix, pheniprazine, phenoxypropazine, pivalylbenzhydrazine, safrazine, caroxazone, minaprine, brofaromine, eprobemide, methylene blue, metralindole, curcumin, harmaline, harmine, amiflamine, befloxatone, cimoxatone, esuprone, sercloremide, tetrindole, CX157; catechol o-methyltransferase (COMT) inhibitors such as entacapone, tolcapone, nebicapone, nitecapone, opicapone; anticholinergics such as benztropine, trihexyphenidyl, clozapine, quetiapine, atropine, biperiden, chlorpheniramine, certain SSRIs (such as citalopram), dicyclomine (dicycloverine), dimenhydrinate, diphenhydramine, doxepin, doxylamine, glycopyrrolate, glycopyrronium, hoscyamine, ipratropium, orphenadrine, oxitropium, oxybutynin, promethazine, propantheline bromide, scopolamine, solifenacin, tolterodine, tiotropium, tricyclic antidepressants (28 compounds with numerous trade names), tropicamide, bupropion, dextromethorphan, doxacurium, hexamethonium, mecamylamine, tubocurarine; and amantadine.

Examples of therapeutic agents for topical dermatitis include creams that control itching and repair the skin (that may contain corticosteroids, and/or calcineurin inhibitors such as tacrolimus, pimecrolimus; drugs to fight the infection (oral or topical antibiotic containing formulations; oral corticosteroids; janus kinase inhibitors such as previously described; and dupilumab.

Examples agents for the treatment of macular degeneration include bevacizumab, ranibizumab, aflibercept, vitamin C, vitamin E, lutein, zeaxantin.

In a particular embodiment, the cosmetical composition, the dietary supplement, the functional food or beberage, the premix, the animal feed, the pet food, or the medical food composition as defined above further comprises one or more nutraceutical ingredient such as vitamins, carotenoids, and flavonoids.

Examples of vitamins include but are not limited to Vitamin A (acetate or palmitate, betacarotene), vitamin B1 (thiamine (aneurine)) (hydrochloride or mononitrate), B2 (riboflavin), vitamin B6 (pyridoxine hydrochloride), vitamin B12 (cobalamin), vitamin B12 (cyanocobalamin), vitamin B12 (mecobalamin), vitamin C (ascorbic acid), nicotinic acid, vitamin D2 (ergo-calciferol), vitamin E (alpha tocopheryl acetate, alpha tocopheryl succinate, alpha tocopherol, $\gamma$-tocopherol), vitamin K (phylloquinone, menadione etc), and nicotinamide riboside.

Examples of carotenoids include, but are not limited to, lutein, lycopene, $\alpha$-carotene, $\beta$-carotene, $\gamma$-carotene, $\beta$-cryptoxanthin, Capsanthin, Zeaxanthin, Astaxanthin.

Examples of flavonoids include, but are not limited to, kaempferol, myricetin, quercetin, rutin, catechin, epicatechin, ECG, gallocatechin, EGC, EGCG, cyanidin, caffeic acid, theaflavin, theaflavin gallate, luteolin, daidzein, genestein, and glycitein.

The compositions of the present invention can be prepared according to methods well known in the state of the art. The appropriate excipients and/or carriers, and their amounts, can readily be determined by those skilled in the art according to the type of formulation being prepared.

All the embodiments disclosed above for the cocrystal of a compound of formula (I) or of formula (II) and a hydrogen bond donor coformer as defined above also applies to the compositions of the invention.

As mentioned above, the third aspect of the invention refers to a cocrystal of a compound of formula (I) or of formula (II) and a hydrogen bond donor coformer as defined above for use as a medicament.

Particularly, the cocrystal of a compound of formula (I) or of formula (II) and a hydrogen bond donor coformer as defined above is for use in the prophylaxis and/or treatment of Vitamin D deficiency, rickets, osteomalacia, osteoporosis, cancer, type1 and type 2 diabetes, hypertension, glucose intolerance, multiple sclerosis, influenza and other diseases mediated by the immune system such as Alzheimer Disease, Parkinson, topical dermatitis, macular degeneration. This aspect could be also formulated as the use of the a cocrystal of a compound of formula (I) or of formula (II) and a hydrogen bond donor coformer as defined above, for the preparation of a medicament for the prophylaxis and/or treatment of Vitamin D deficiency, rickets, osteomalacia, osteoporosis, cancer, type1 and type 2 diabetes, hypertension, glucose intolerance, multiple sclerosis, influenza and other diseases mediated by the immune system such as Alzheimer Disease, Parkinson, topical dermatitis, macular degeneration. It also relates to a method for the prophylaxis and/ treatment of Vitamin D deficiency, rickets, osteomalacia, osteoporosis, cancer, type1 and type 2 diabetes, hypertension, glucose intolerance, multiple sclerosis, influenza and other diseases mediated by the immune system such as Alzheimer Disease, Parkinson, topical dermatitis, macular degeneration, wherein the method comprises administering to said mammal an effective amount of the cocrystal of a compound of formula (I) or of formula (II) and a hydrogen bond donor coformer as defined above, together with one or more acceptable excipients or carriers.

Throughout the description and claims the word "comprise" and variations of the word, are not intended to

exclude other technical features, additives, components, or steps. Furthermore, the word "comprise" encompasses the case of "consisting of".

The following examples and drawings are provided by way of illustration, and they are not intended to be limiting of the present invention. Furthermore, the present invention covers all possible combinations of particular and preferred embodiments described herein.

## Examples

### General considerations

Ergocalciferol, cholecalciferol, calcifediol, ercalcidiol, calcitriol, ercalcitriol and alfacalcidol 7-dehydrocholesterol, are commercially available.

Powder X-Ray diffraction (PXRD) analyses were performed by sandwiching the powder samples between polyester films of 3.6 microns of thickness analysed in a PANalytical X'Pert PRO MPD $\theta/\theta$ powder diffractometer of 240 millimetres of radius, in a configuration of convergent beam with a focalizing mirror and a flat sample transmission geometry, in the following experimental conditions: Cu K$\alpha$ radiation ($\lambda$ = 1.5418 Å); Work power: 45 kV and 40 mA; Incident beam slits defining a beam height of 0.4 millimetres; Incident and diffracted beam 0.02 radians Soller slits; PIXcel detector: Active length = 3.347 °; 2$\theta$/$\theta$ scans from 2 to 40° 2$\theta$ with a step size of 0.026° 2$\theta$ and a measuring time of 76 seconds per step.

Example 1.- Preparation of Form $VD_3$:resorcinol cocrystal polymorph A

$VD_3$ (500 mg, 1.30 mmol) and resorcinol (143 mg, 1.30 mmol) were stirred in pentane (3.0 mL). Then, acetone (2.0 mL) was added until total dissolution. The clear solution was air-opened and kept at 4-6 °C. A yellow glassy oil was observed after 23 days and it was dried under vacuum until a solid crystallized.

Example 2.- Preparation of Form $VD_3$:resorcinol cocrystal polymorph B

A concentrated solution of resorcinol in ACN was prepared (300 mg in 0.1 mL). A suspension was prepared by adding $VD_3$ (150 mg, 0.39 mmol) and it was stirred overnight at room temperature. A clear solution was obtained the day after and it was air-opened and kept at 4-6 °C. A solid was observed after 9 days and it was filtered and dried under vacuum.

Example 3.- Preparation of a cocrystal of Form $VD_2$:resorcinol

Concentrated solution of $VD_2$ in MEK was prepared (100 mg in 0.1 mL). Resorcinol was added until a suspension was observed. The suspension was stirred overnight at room temperature. The solid was filtered and dried under vacuum.

Example 4.- Preparation of a cocrystal of $VD_3$:orcinol

$VD_3$ (378.1 mg, 0.983 mmol) and orcinol (121.9 mg, 0.983 mmol) were stirred in hexane (2.0 mL) overnight at room temperature. The solid was filtered and dried under vacuum.

Example 5.- Preparation of Form $VD_2$:orcinol cocrystal polymorph A

$VD_2$ (500 mg, 1.261 mmol) was stirred in toluene (0.65 mL) during 30 minutes until total dissolution. Orcinol (104.3 mg, 0.839 mmol) was added and a suspension was observed. The suspension was stirred at room temperature for 3 days. The solid was filtered and dried under vacuum.

Example 6.- Preparation of Form $VD_2$:orcinol cocrystal polymorph B

Form $VD_2$:orcinol cocrystal polymorph B was obtained by heating of solid of $VD_2$:orcinol cocrystal polymorph A between 70 °C and 80 °C in DSC or XRPD temperature variable experiment.

Example 7.- Preparation of a cocrystal of $VD_2$:beta-sitosterol

$VD_2$ (200 mg, 0.504 mmol) was dissolved in ACN (25 mL) at 50 °C. The solution was cooled down at room temperature and no solid was observed. Beta-sitosterol (83 mg, 0.200 mmol) was added to form a suspension. The

suspension was stirred at room temperature overnight. The solid was filtered and dried under vacuum.

Comparative Example 1.- Preparation of a cocrystal of $VD_2$:propionic acid

$VD_2$ (50 mg, 0.126 mmol) and propionic acid (0.05 mL) were grinded at 30 Hz during 15 minutes at room temperature. A cocrystal of $VD_2$:propionic acid was obtained. The cocrystal was characterized by exhibiting in the X-ray powder diffractogram a pattern of peaks, expressed in 2 theta units in degrees (Cu-$K_a$ radiation, $\lambda$ = 1.5418 Å), which is shown in Table 8.

| Pos. [°2Th.] | Rel. Int. [%] |
|---|---|
| 6.0043 | 15.16 |
| 6.9213 | 7.62 |
| 9.1577 | 16.05 |
| 11.3523 | 8.5 |
| 12.0067 | 1.92 |
| 12.4096 | 9.51 |
| 12.7661 | 6.55 |
| 13.8106 | 2.08 |
| 15.0963 | 25.17 |
| 15.3395 | 59.85 |
| 16.1007 | 79.47 |
| 16.5378 | 18.43 |
| 16.9253 | 100 |
| 18.3533 | 9.86 |
| 19.3294 | 12.96 |
| 19.5146 | 11.18 |
| 19.8962 | 1.55 |
| 20.4662 | 8.02 |
| 20.8186 | 2.31 |
| 21.049 | 4.09 |
| 21.6802 | 7.23 |
| 21.9149 | 7.3 |
| 22.1914 | 4.41 |
| 22.7132 | 7.56 |
| 24.0852 | 7.48 |
| 24.512 | 4.9 |
| 25.1064 | 8.46 |
| 26.8357 | 4.28 |
| 29.9381 | 4.29 |

The cocrystal of $VD_2$:propionic acid of the present disclosure may be further characterized by an X-ray diffractogram as in Figure 8.

Example 8.- Photostability of the new $VD_2$ and $VD_3$ cocrystals versus $VD_2$/$VD_3$ cocrystal

In order to compare the photostability of the new $VD_2$ and $VD_3$ cocrystals with the one of $VD_2$/$VD_3$ (form A) cocrystal and of pure $VD_2$ and $VD_3$, the different samples were subjected to accelerated storage conditions at 23 °C and illumination with 5000 lx for 90 days. $VD_2$/$VD_3$ (form A) cocrystal was prepared according to the procedure described in Wang et al. Chem. Commun., 2016, 52, 3572.

The samples under study were periodically analyzed by HPLC as follows:

Determination of vitamin $D_2$/$D_3$ content in each sample was performed by HPLC (Agilent 1100 Series HPLC) equipped with a quaternary bomb (G1311A), a degasifier (G1322A), an injector (G1329A) and diode-array detector (G1315B) using a Kinetex C18 column (100 mm × 4.6 mm, 2.6 $\mu$m) and a mobile phase (A: Water 0,1% formic acid, and B:

Acetonitrile) with a flow rate of 0.8 mL min-1 and an isocratic at 95% B (8 min analysis time) gradient and it was acquired at a wavelength of 270 nm.

Figures 9 and 10 show the decrease in $VD_2$ and $VD_3$ content of new $VD_2$ and $VD_3$ cocrystals compared to $VD_2$/$VD_3$ (form A) cocrystal and pure $VD_2$ and $VD_3$, respectively, during storage at 23 °C with an illuminance of 5000 lx.

Example 9.- Stability under stress conditions

*Comparative Vitamin $D_3$ ($VD_3$) vs Vitamin $D_3$ cocrystals under different stress conditions*

Similarly as above, the stability of the $VD_3$:orcinol cocrystal was studied and compared to stability of $VD_3$ (Form A) under stress conditions (40 °C - 75% RH). The samples were stored under these conditions inside a Series FD Binder chamber and they were analyzed periodically by PXRD. A saturated solution of sodium chloride (75%) was used to control relative humidity.

As shown in Table 3, $VD_3$ (Form A) exhibited a very low stability under stress conditions since it converted into a liquid phase in five days. However, $VD_3$:orcinol cocrystal exhibited a very high stability under stress conditions since it remained as the same starting solid form after 230 days.

Table 3

| Time (days) | 40 °C-75%RH | |
|---|---|---|
| | **$VD_3$** | **$VD_3$:orcinol** |
| 0 | Form A | $VD_3$:orcinol |
| 5 | liquid | $VD_3$:orcinol |
| 14 | - | $VD_3$:orcinol |
| 30 | - | $VD_3$:orcinol |
| 60 | - | $VD_3$:orcinol |
| 150 | - | $VD_3$:orcinol |
| 230 | - | $VD_3$:orcinol |

*Comparative of Vitamin $D_2$ ($VD_2$) vs Vitamin $D_2$ cocrystals under different stress conditions*

The stability of the $VD_2$ cocrystal with beta-sitosterol and $VD_2$ cocrystal with orcinol was studied and compared to the stability of $VD_2$ (Form A) under two stress conditions. The samples were stored under both stress conditions inside a Series FD Binder chamber and they were analyzed periodically by PXRD. The two stress conditions were the following:

- condition A: 25 °C and 57% relative humidity;

- condition B: 40 °C and 75% relative humidity.

A saturated solution of sodium bromide salt (for 57% RH) and a saturated solution of sodium chloride (for 75% RH) were used to control relative humidity.

Table 1 and 2 show the evolution of the different solid forms over time.

Table 1

| Time (days) | 25 °C-57%RH | | |
|---|---|---|---|
| | **$VD_2$** | **$VD_2$: beta-sitosterol** | **$VD_2$: orcinol** |
| 0 | Form A | $VD_2$: beta-sitosterol | $VD_2$:orcinol |
| 7 | - | - | |
| 14 | - | - | $VD_2$:orcinol |
| 25 | Form A with presence of amorphous phase' | $VD_2$: beta-sitosterol | |
| 50 | - | - | $VD_2$:orcinol |
| 130 | | - | $VD_2$:orcinol |
| 210 | Form A with presence of amorphous phase2 | $VD_2$: beta-sitosterol | - |
| 370 | Amorphous phase | $VD_2$: beta-sitosterol | - |

Table 2

| Time (days) | 40 °C-75%RH | | |
|---|---|---|---|
| | $VD_2$ | $VD_2$: beta-sitosterol | $VD_2$: orcinol |
| 0 | Form A | $VD_2$: beta-sitosterol | $VD_2$:orcinol |
| 7 | - | | - |
| 14 | - | | $VD_2$:orcinol |
| 25 | Form A with presence of amorphous phase' | $VD_2$: beta-sitosterol | - |
| 50 | - | | $VD_2$:orcinol |
| 130 | - | | $VD_2$:orcinol with presence of Form A |
| 210 | Liquid phase | Amorphous | - |
| 370 | - | Amorphous | - |

[1] The solid has a brown colour and its PXRD shows the characteristic halo of amorphous phase.
[2] The solid shows the amorphous solid as the major solid phase, with traces of crystalline $VD_2$ (form A) in the mixture and the sample shows a brown colour.

$VD_2$ (Form A) exhibited a very low stability under both stress conditions since it started to convert into an amorphous phase in 25 days. However, $VD_2$:orcinol cocrystal exhibited a very high stability under both stress conditions since it remained as the same starting solid form after 130 days under 25 °C and 57% of relative humidity and 50 days under 40 °C and 75% of relative humidity. On the other hand, $VD_2$:beta-sitosterol cocrystal exhibited also a very high stability under both stress conditions since it remained as the same starting solid form after 370 days under 25 °C and 57% of relative humidity and 25 days under 40 °C and 75% of relative humidity.

Example 10. Dissolution rate of the cocrystal of $VD_2$ and $VD_3$ in FaSSIF v2 medium

*1. Molar Extinction Coefficient (MEC) determination in FaSSIF v2 medium*

Molar extinction coefficients of $VD_2$, $VD_3$ and of the coformer were determined by UV-metric titration using a GlpKaTM titrator (Sirius Analytical Instruments, UK). Briefly, a 10 mM stock solution of sample was prepared in DMSO. 50 µL of sample stock solution and 0.25 mL of a 15 mM potassium phosphate buffer were added to 10 mL of a 0.15 M KCl solution, which in turn contained 17.9 mg of FaSSIF v2 powder. The pH of the sample solution was adjusted to 2 with 0.5 M HCl before starting the titration, and then the titration was done using 0.5 M KOH up to pH 12. The UV absorption spectra (between 250 and 450 nm) of the solution were recorded at each titrant addition by a fiber optic dip-probe. The collected data were refined through the RefinamentPro software, and the pKa values and Molar Extinction Coefficients obtained by Target Factor Analysis.

*2. Dissolution Rate experiments*

- Tablet production: tablets of 3 mm diameter were done using a manual hydraulic tablet press (Applied Measurements Ltd, UK). The applied pressure was 100 Kg for 2 min. From 6 to 10 mg of each solid form, vitamin or cocrystal, were weighted. The total exposed surface area was 0.5 $cm^2$.
- Medium: FaSSIF v2 (pH 6.5), with corresponding amount of phosphoric (28.4 mM) acid instead of maleic acid.
- Dissolution tests were performed with a small-scale dissolution assay installed in a GlpKaTM titrator (Sirius Analytical Instruments, UK).
- Dissolution time and temperature: 120 minutes and 25 °C.
- Procedure: 15 mL of FaSSIF v2 was added into the sample vial containing the tablet. Spectra collection started immediately after. Spectra were recorded every 30 seconds between 250 and 450 nm through a Sirius D-PAS

spectrometer with a bifurcated optic fibre dip probe (Hellma Analytics). The medium was stirred at a constant rate.

*3. UV-vis Quantification*

The concentration of cholecalciferol ($VD_3$) or ergocalciferol ($VD_2$) in solution at each time point is determined from the spectroscopic data by applying the Beer-Lambert law, using previously determined molar extinction coefficients of the pure APIs and the coformers. Spectrum regions where signal is saturated (A>1.5) or presenting medium interferences were discarded. Then, the concentration data were converted into absolute sample quantities, and used to generate the graphs showing the sample quantity vs. time.

*4. Determination of the dissolution rate*

Dissolution rate is obtained through the fit of the first order Noyes-Whitney exponential equation to the data:

$$[X]t = S(1 - e{-}kd(t{-}t0))$$

In this equation, $[X]_t$ is the weight in grams of compound in solution at the experiment time (min); *S* is the extrapolated solubility (g) of the vitamin; $k_d$ is the rate constant for dissolution ($min^{-1}$); and $t_0$ (min) is a term allowing for a temporal offset. Results are calculated using a refinement process in which *S*, $k_d$ and $t_0$ are varied in order to minimize the root mean square deviation between the modelled concentrations and the measured concentrations. The dissolution rate (g $min^{-1}$) is given by the product $k_dS$ (T. Gravestock et al., Anal. Methods, 2011, 3, 560). Results are shown in Table 3.

Table 3

| Solid form | dissolution rate (nmol/min) |
|---|---|
| $VD_2$ | 0.74 ± 0.23 |
| $VD_3$ | 0.58 ± 0.14 |
| $VD_3$ - $VD_2$ | 0.63 ± 0.21 |
| $VD_3$ - resorcinol | 0.94 ± 0.22 |

The comparison between dissolution rate curves of the solid forms listed in Table 3 is shown in Figure 11.

It can be seen that $VD_3$-resorcinol dissolved faster than $VD_2$, $VD_3$, or $VD_2$-$VD_3$.

Example 10.- Stability of *Vitamin $D_3$ ($VD_3$) vs Vitamin $D_3$:resorcinol cocrystal in a premix*

The stability of the $VD_3$:resorcinol cocrystal mixed in a premix that was a standard mineral-vitamin mix containing choline was studied under normal and high environmental temperature/humidity and compared to the stability of commercial $VD_3$ for animal nutrition (Orffa D3 500) in the same premix under de same conditions.

*Environmental conditions*

- 25 °C and 57% humidity (temperature controlled chambers with a saturated solution of NaBr to maintain humidity at 57%);
- 40 °C and 75% humidity (temperature controlled chambers with a saturated solution of NaCl to maintain humidity at 75%).

The tested $VD_3$:resorcinol and $VD_3$ were mixed in a standard vitamin and mineral mix containing components shown in Table 4.

Table 4

| Nutritional additive | mg/kg |
|---|---|
| Copper (copper sulfate pentahydrated) | 4,350 |

(continued)

| Nutritional additive | mg/kg |
|---|---|
| Zinc (Zinc Sulfate monohydrate) | 17,920 |
| Manganese (Magnesium sulfate monohydrate) | 5,394 |
| Iodine (Potassium iodine) | 137 |
| Selenium (Sodium Selenite) | 91 |
| Cobalt (Cobalt Acetate tetrahydrate) | 47 |
| Zinc (Zinc-AA quelate, Hydrated) | 5,000 |

The mineral mix was supplemented to contain 750,000 UI/kg Vitamin A, 5,000 mg/kg vitamin E and 12.5% choline chloride (choline chloride, vegetal coating, 60%). $VD_3$ final content was 1,600,000 UI/Kg (both for the cocrystal $VD_3$:resorcinol and for the commercial $VD_3$.

*Procedures*

Samples (2 g) were weighted in 50 mL plastic tubes and incubated in the experimental conditions (see above). Samples for each individual time were incubated in quadruplicates to account for potential variation. Samples were prepared to be retrieved from incubators at 2, 4, 8 and 12 weeks. After each time, samples were processed and analyzed immediately (no storage).

Samples were subjected to an extraction process done in the incubation tube with the full sample to avoid sub-sampling problems. Extracted supernatant was analysed for vitamin D using and HPLC (Model 1260 Series, Agilent, Darmstadr, Germany) coupled with a MS-QQQ (Model G6420, Agilent) against a calibrated curve using a standard vitamin $D_3$. Results are reported as a percent degradation compared with the original Vitamin concentration.

*Results*

Temperature and humidity were maintained within the pre-planned conditions. Concentrations of $VD_3$ for the Control and of $VD_3$:resorcinol are presented in Figure 12. $VD_3$ was more stable at 25°C/57%H compared with 40°C/75%H, as expected. At 25°C/57%H $VD_3$:resorcinol was more stable than $VD_3$. At 40°C/75%H, both products had a sharp reduction in stability, but $VD_3$:resorcinol has a better performance than commercial $VD_3$.It should be noted that the mineral mix contained high levels of choline chloride which is highly aggressive towards other nutrients, including vitamins.

These results indicate a consistent evolution of the degradation kinetics of $VD_3$ and of $VD_3$:resorcinol and at the different conditions. In both conditions, $VD_3$:resorcinol remains more stable than $VD_3$.

**Citation List**

1. Wang, JR et al. 2013 "Stabilizing vitamin D by conformationally selective co-crystallization" Chem. Commun., 2014, Vol. 50, pp. 855-858.

2. Wang J.R. et al. " Drug-drug co-crystallization presents a new opportunity for the development of stable vitamins" Chem. Commun., 2016, Vol. 52, pp. 3572-3575.


**Claims**

**1.** A cocrystal of a compound of formula (I) or of a compound formula (II)

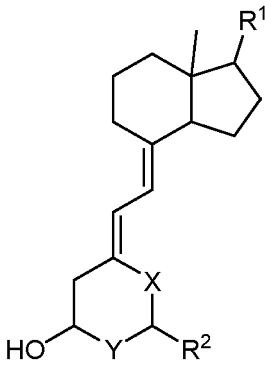 (I) 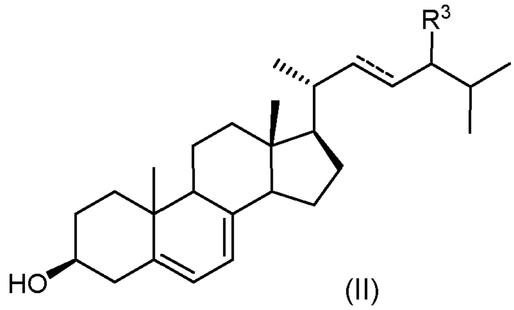 (II)

wherein

X and Y are independently selected from $-CH_2-$ and $-C(CH_2)-$, provided that al least one of X or Y is $-CH_2-$;
$R^1$ is $-CHCH_3-Z_m-W_n-(CH_2)_o-T_p-S$, wherein
Z is O, and m is 0 or 1;
W is $R^4CH---CHR^5$, wherein either $R^4$ and $R^5$ are H and the dashed line indicates that there is a single bond, or $R^4$ and $R^5$ together are forming a bond and the dashed line indicates that there is a double bond, and n is 0 or 1;
o is 0, 1 or 3;
T is selected from the group consisting of $-CHR^6-$ and -C(O), wherein $R^6$ is -OH or $-CH_3$, and p is 0 or 1;
S is selected from the group consisting of H, $(C_1-C_3)$ alkyl optionally substituted by -OH, $(C_1-C_3)$ haloalkyl optionally substituted by -OH, cyclopropyl, and

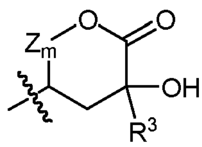 ;

and
$R^2$ is -H or -OH;
$R^3$ is H or $CH_3$; and
the dashed line in formula (II) indicates a single or a double bond;
and a hydrogen bond donor coformer which is a phenolic compound.

**2.** The cocrystal of claim 1, wherein $R^1$ is selected from the group consisting of

$-CHCH_3-(CH_2)_3-CH(CH_3)_2$,
$-CHCH_3-(CH_2)_5-C(CH_3)_2OH$,
$-CHCH_3-(CH_2)_3-C(CH_3)_2OH$,
$-CHCH_3-(CH_2)_2-CH_2OH-C(CH_3)_2OH$,
$-CHCH_3-(CH_2)_2-CH_2OH-CH(CH_3)_2$,
$-CHCH_3-(CH_2)_2-CO-C(CH_3)_2OH$,
$-CHCH_3-(CH_2)_2-CO-CH(CH_3)_2$,
$-CHCH_3-CH=CH-CHCH_3-CH(CH_3)_2$,
$-CHCH_3-CH=CH-CHCH_3-C(CH_3)_2OH$,
$-CHCH_3-CH_2-CH_3$,
$-CHCH_3-(CH_2)_3-C(CF_3)_2OH$,
$-CHCH_3-O-(CH_2)_2-C(CH_3)_2OH$,
$-CHCH_3-CH=CH-CHOH-(CH)_3$,

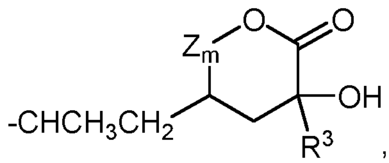

wherein Z, $R^3$ and m are as defined in claim 1.

**3.** The cocrystal of claim 2, wherein the compound of formula (I) is selected from the group consisting of ergocalciferol, cholecalciferol, calcifediol, ercalcidiol, calcitriol, ercalcitriol, alfacalcidol, tacalcitol, becocalcidiol, doxercalciferon, paricalcitol, calcipotriene, calcitroic acid, calcitetrol, 24-oxo-1alpha,25-dihydroxyvitamin D3, calcitriol-26,23-lactone, 1a,25-dihydroxy-cholecalciferol-26,23-peroxylactone, 26,26,26,27,27,27-hexafluoro-1alpha,25-dihydroxycholecalciferol, 24-hydroxyvitamin D3, 1alpha-hydroxy-24-oxocholecalciferol, calcifediol lactone, (23S,25R)-25-hydroxycholecalciferol-26,23-peroxylactone, 1,25-dihydroxy-24-oxo-vitamin D3, 24,25- dihydroxycholecalciferol, 25-hydroxy-24-oxo-cholecalciferol, and maxacalcitol; and the compound of formula (II) is selected from ergosterol and 7-dehidrocholesterol.

**4.** The cocrystal of claim 3, which is a cocrystal of a compound of formula (I) selected from the group consisting of ergocalciferol, cholecalciferol, calcifediol, ercalcidiol, calcitriol, ercalcitriol, and alfacalcidol; and a phenolic compound.

**5.** The cocrystal according to any one of claims 1 to 4, wherein the phenolic compound is orcinol or resorcinol.

**6.** The cocrystal according to claim 3, which is cocrystal of vitamin $D_3$ and resorcinol polymorph A, **characterized by** having an X-ray powder diffractogram that comprises characteristic peaks at approximately 3.6, 11.2, 14.6, 16.2, and 17.0 $\pm$ 0.3 degrees 2 theta (Cu-$K_a$ radiation, $\lambda$ = 1.5418 Å).

**7.** The cocrystal according to claim 3, which is cocrystal of vitamin $D_3$ and resorcinol polymorph B, **characterized by** having an X-ray powder diffractogram that comprises characteristic peaks at approximately 3.5, 9.4, 11.2, 15.7, and 17.5 $\pm$ 0.3 degrees 2 theta (Cu-$K_a$ radiation, $\lambda$ = 1.5418 Å).

**8.** The cocrystal according to claim 3, which is cocrystal of vitamin $D_2$ and resorcinol, **characterized by** having an X-ray powder diffractogram that comprises characteristic peaks at approximately 3.4, 11.1, 15.5, 16.3, and 17.0 $\pm$ 0.3 degrees 2 theta (Cu-$K_a$ radiation, $\lambda$ = 1.5418 Å).

**9.** The cocrystal according to claim 3, which is cocrystal of vitamin $D_3$ and orcinol, **characterized by** having an X-ray powder diffractogram that comprises characteristic peaks at approximately 3.3, 9.9, 11.4, 16.0 and 17.7 $\pm$ 0.3 degrees 2 theta (Cu-$K_a$ radiation, $\lambda$ = 1.5418 Å).

**10.** The cocrystal according to claim 3, which is cocrystal of vitamin $D_2$ and orcinol polymorph A, **characterized by** having an X-ray powder diffractogram that comprises characteristic peaks at approximately 3.2, 9.8, 11.2, 15.7, and 17.5 $\pm$ 0.3 degrees 2 theta (Cu-$K_a$ radiation, $\lambda$ = 1.5418 Å).

**11.** The cocrystal according to claim 3, which is cocrystal of vitamin $D_2$ and orcinol polymorph B, **characterized by** having an X-ray powder diffractogram that comprises characteristic peaks at approximately 3.1, 9.7, 14.6, 17.0, and 17.3 $\pm$ 0.3 degrees 2 theta (Cu-$K_a$ radiation, $\lambda$ = 1.5418 Å).

**12.** A composition comprising an effective amount of the cocrystal of a compound of formula (I) or of formula (II) and a hydrogen bond donor coformer as defined in any one of claims 1 to 11 together with one or more appropriate acceptable excipients or carriers.

**13.** The composition according to claim 12, which is a pharmaceutical or veterinary composition, a cosmetical composition, a dietary supplement including functional food and functional beverages, a medical food, a premix, an animal feed, or a pet food.

**14.** A personal care product product comprising the composition as defined in claim 12.

**15.** A cocrystal of a compound of formula (I) or of formula (II) and a hydrogen bond donor coformer as defined in any one of claims 1 to 12 for use as a medicament.

## Patentansprüche

**1.** Ein Cokristall aus einer Verbindung der Formel (I) oder einer Verbindung der Formel (II)

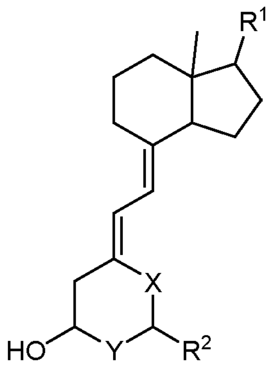
(I)

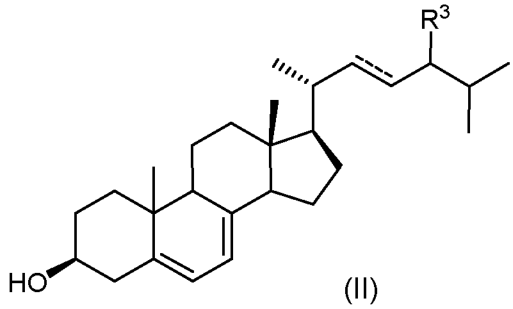
(II)

wobei

X und Y unabhängig voneinander aus $-CH_2-$ und $-C(CH_2)-$ ausgewählt sind, unter der Voraussetzung, dass mindestens eines von X oder Y $-CH_2-$ ist;
$R^1$ $-CHCH_3-Z_m-W_n-(CH_2)_o-T_p-S$ ist, wobei
Z = O ist und m = 0 oder 1 ist;
W $R^4CH$- - -$CHR^5$ ist, wobei entweder $R^4$ und $R^5$ = H sind und die gestrichelte Linie angibt, dass eine Einfachbindung vorliegt, oder $R^4$ und $R^5$ zusammen eine Bindung bilden und die gestrichelte Linie angibt, dass eine Doppelbindung vorliegt, und n = 0 oder 1 ist;
o = 0, 1 oder 3 ist;
T ausgewählt ist aus der Gruppe bestehend aus $-CHR^6-$ und -C(O), wobei $R^6$ -OH oder $-CH_3$ ist und p = 0 oder 1 ist;
S ausgewählt ist aus der Gruppe bestehend aus H, $(C_1-C_3)$-Alkyl, das gegebenenfalls durch -OH substituiert ist, $(C_1-C_3)$-Halogenalkyl, das gegebenenfalls durch -OH substituiert ist, Cyclopropyl und

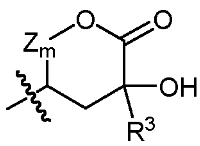 ; und

$R^2$ -H oder -OH ist;
$R^3$ H oder $CH_3$ ist; und
die gestrichelte Linie in Formel (II) eine Einfach- oder eine Doppelbindung angibt;
und einem Wasserstoffbrückendonor-Coformer, der eine phenolische Verbindung ist.

**2.** Der Cokristall von Anspruch 1, wobei $R^1$ ausgewählt ist aus der Gruppe bestehend aus

$-CHCH_3-(CH_2)_3-CH(CH_3)_2$,
$-CHCH_3-(CH_2)_5-C(CH_3)_2OH$,
$-CHCH_3-(CH_2)_3-C(CH_3)_2OH$,
$-CHCH_3-(CH_2)_2-CH_2OH-C(CH_3)_2OH$,
$-CHCH_3-(CH_2)_2-CH_2OH-CH(CH_3)_2$,
$-CHCH_3-(CH_2)_2-CO-C(CH_3)_2OH$,
$-CHCH_3-(CH_2)_2-CO-CH(CH_3)_2$,
$-CHCH_3-CH=CH-CHCH_3-CH(CH_3)_2$,

$-CHCH_3-CH=CH-CHCH_3-C(CH_3)_2OH$,
$-CHCH_3-CH_2-CH_3$,
$-CHCH_3-(CH_2)_3-C(CF_3)_2OH$,
$-CHCH_3-O-(CH_2)_2-C(CH_3)_2OH$,
$-CHCH_3-CH=CH-CHOH-(CH)_3$,

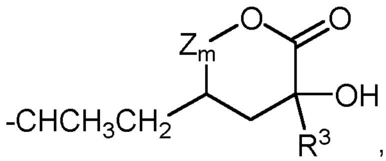

wobei Z, $R^3$ und m wie in Anspruch 1 definiert sind.

3. Der Cocrystal von Anspruch 2, wobei die Verbindung der Formel (I) ausgewählt ist aus der Gruppe bestehend aus Ergocalciferol, Cholecalciferol, Calcifediol, Ercalcidiol, Calcitriol, Ercalcitriol, Alfacalcidol, Tacalcitol, Becocalcidiol, Doxercalciferon, Paricalcitol, Calcipotrien, Calcitrosäure, Calcitetrol, 24-Oxo-1alpha,25-Dihydroxyvitamin D3, Calcitriol-26,23-Lacton, 1a,25-Dihydroxycholecalciferol-26,23-Peroxylacton, 26,26,26,27,27-Hexafluor-1alpha,25-Dihydroxycholecalciferol, 24-Hydroxyvitamin D3, 1alpha-Hydroxy-24-Oxocholecalciferol, Calcifediol-Lacton, (23S,25R)-25-Hydroxycholecalciferol-26,23-Peroxylacton, 1,25-Dihydroxy-24-Oxo-vitamin D3, 24,25-Dihydroxycholecalciferol, 25-Hydroxy-24-Oxo-cholecalciferol und Maxacitol; und die Verbindung der Formel (II) ausgewählt ist aus Ergosterol und 7-Dehidrocholesterol.

4. Der Cokristall von Anspruch 3, der ein Cokristall aus einer Verbindung der Formel (I) ist, die ausgewählt ist aus der Gruppe bestehend aus Ergocalciferol, Cholecalciferol, Calcifediol, Ercalcidiol, Calcitriol, Ercalcitriol und Alfacalcidol; und einer phenolischen Verbindung.

5. Der Cokristall nach einem der Ansprüche 1 bis 4, wobei die phenolische Verbindung Orcin oder Resorcin ist.

6. Der Cokristall nach Anspruch 3, der Polymorph A des Cokristalls von Vitamin $D_3$ und Resorcin ist, **dadurch gekennzeichnet, dass** er ein Röntgenpulverdiffraktogramm hat, das charakteristische Peaks bei etwa 3,6, 11,2, 14,6, 16,2 und 17,0 $\pm$ 0,3 Grad 2 Theta (Cu-$K_a$ -Strahlung, $\lambda$ = 1,5418 Å) umfasst.

7. Der Cokristall nach Anspruch 3, der Polymorph B des Cokristalls von Vitamin $D_3$ und Resorcin ist, **dadurch gekennzeichnet, dass** er ein Röntgenpulverdiffraktogramm hat, das charakteristische Peaks bei etwa 3,5, 9,4, 11,2, 15,7 und 17,5 $\pm$ 0,3 Grad 2 Theta (Cu-$K_a$ - Strahlung, $\lambda$ = 1,5418 Å) umfasst.

8. Der Cokristall nach Anspruch 3, der ein Cokristall von Vitamin $D_2$ und Resorcin ist, **dadurch gekennzeichnet, dass** er ein Röntgenpulverdiffraktogramm hat, das charakteristische Peaks bei etwa 3,4, 11,1, 15,5, 16,3 und 17,0 $\pm$ 0,3 Grad 2 Theta (Cu-$K_a$ -Strahlung, $\lambda$ = 1,5418 Å) umfasst.

9. Der Cokristall nach Anspruch 3, welcher ein Cokristall von Vitamin $D_3$ und Orcin ist, **dadurch gekennzeichnet, dass** er ein Röntgenpulverdiffraktogramm hat, das charakteristische Peaks bei etwa 3,3, 9,9, 11,4, 16,0 und 17,7 $\pm$ 0,3 Grad 2 Theta (Cu-$K_a$ - Strahlung, $\lambda$ = 1,5418 Å) umfasst.

10. Der Cokristall nach Anspruch 3, der Polymorph A des Cokristalls von Vitamin $D_2$ und Orcin ist, **dadurch gekennzeichnet, dass** er ein Röntgenpulverdiffraktogramm hat, das charakteristische Peaks bei etwa 3,2, 9,8, 11,2, 15,7 und 17,5 $\pm$ 0,3 Grad 2 Theta (Cu-$K_a$ - Strahlung, $\lambda$ = 1,5418 Å) umfasst.

11. Der Cokristall nach Anspruch 3, der Polymorph B des Cokristalls von Vitamin $D_2$ und Orcin ist, **dadurch gekennzeichnet, dass** er ein Röntgenpulverdiffraktogramm hat, das charakteristische Peaks bei etwa 3,1, 9,7, 14,6, 17,0 und 17,3 $\pm$ 0,3 Grad 2 Theta (Cu-$K_a$ - Strahlung, $\lambda$ = 1,5418 Å) umfasst.

12. Eine Zusammensetzung umfassend eine wirksame Menge des Cokristalls aus einer Verbindung der Formel (I) oder der Formel (II) und einem Wasserstoffbrückendonorbildner, wie in einem der Ansprüche 1 bis 11 definiert, zusammen mit einem oder mehreren geeigneten akzeptablen Hilfsstoffen oder Trägersubstanzen.

**13.** Die Zusammensetzung nach Anspruch 12, welche eine pharmazeutische oder veterinärmedizinische Zusammensetzung, eine kosmetische Zusammensetzung, ein Nahrungsergänzungsmittel einschließlich funktioneller Lebensmittel und funktioneller Getränke, ein medizinisches Lebensmittel, eine Vormischung, ein Tierfutter oder ein Haustierfutter ist.

**14.** Ein Körperpflegeprodukt umfassend die Zusammensetzung wie in Anspruch 12 definiert.

**15.** Ein Cokristall aus einer Verbindung der Formel (I) oder der Formel (II) und einem Wasserstoffbrückendonor-Coformer wie in einem der Ansprüche 1 bis 12 definiert zur Verwendung als Medikament.

## Revendications

**1.** Un cocristal d'un composé de formule (I) ou d'un composé de formule (II)

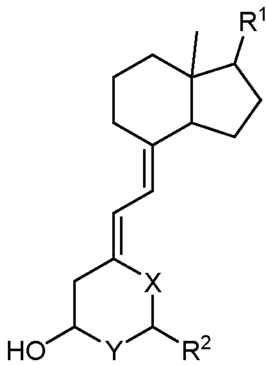 (I) 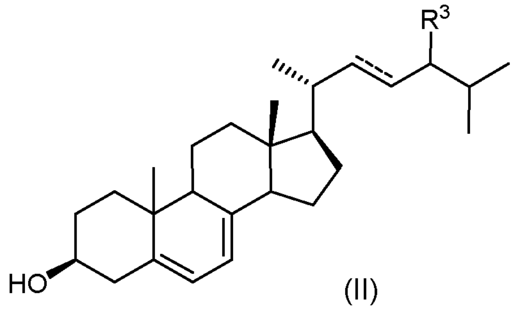 (II)

où

X et Y sont choisis indépendamment parmi $-CH_2-$ et $-C(CH_2)-$, à condition qu'au moins l'un de X ou Y soit $-CH_2-$ ;
$R^1$ est $-CHCH_3-Z_m-W_n-(CH_2)_o-T_p-S$, où
Z est O, et m est 0 ou 1 ;
West $R^4CH$- - -$CHR^5$, où soit $R^4$ et $R^5$ sont H et la ligne en pointillés indique qu'il y a une liaison simple, soit $R^4$ et $R^5$ forment ensemble une liaison et la ligne en pointillés indique qu'il y a une double liaison, et n est 0 ou 1 ;
o est 0, 1 ou 3 ;
T est choisi dans le groupe constitué par $-CHR^6-$ et -C(O), où $R^6$ est -OH ou $-CH_3$, et p est 0 ou 1 ;
S est choisi dans le groupe constitué par H, alkyle en $(C_1-C_3)$ éventuellement substitué par -OH, haloalkyle en $(C_1-C_3)$ éventuellement substitué par -OH, cyclopropyle, et

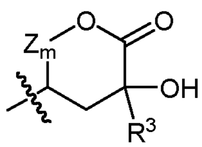 ; et

$R^2$ est -H ou -OH ;
$R^3$ est H ou $CH_3$; et
la ligne en pointillés dans la formule (II) indique une liaison simple ou double ;
et un coformateur donneur de liaison hydrogène qui est un composé phénolique.

**2.** Le cocristal de la revendication 1, dans lequel $R^1$ est choisi dans le groupe constitué par

$-CHCH_3-(CH_2)_3-CH(CH_3)_2$,
$-CHCH_3-(CH_2)_3-C(CH_3)_2OH$,
$-CHCH_3-(CH_2)_3-C(CH_3)_2OH$,
$-CHCH_3-(CH_2)_2-CH_2OH-C(CH_3)_2OH$,

$-CHCH_3-(CH_2)_2-CH_2OH-CH(CH_3)_2$,
$-CHCH_3-(CH_2)_2-CO-C(CH_3)_2OH$,
$-CHCH_3-(CH_2)_2-CO-CH(CH_3)_2$,
$-CHCH_3-CH=CH-CHCH_3-CH(CH_3)_2$,
$-CHCH_3-CH=CH-CHCH_3-C(CH_3)_2OH$,
$-CHCH_3-CH_2-CH_3$,
$-CHCH_3-(CH_2)_3-C(CF_3)_2OH$,
$-CHCH_3-O-(CH_2)_2-C(CH_3)_2OH$,
$-CHCH_3-CH=CH-CHOH-(CH)_3$,

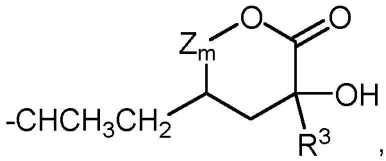
,

où Z, $R^3$ et m sont tels que définis dans la revendication 1.

**3.** Le cocristal de la revendication 2, dans lequel le composé de formule (I) est choisi dans le groupe constitué par l'ergocalciférol, le cholécalciférol, le calcifédiol, l'ercalcidiol, le calcitriol, l'ercalcitriol, l'alfacalcidol, le tacalcitol, le bécocalcidiol, le doxercalciférol, le paricalcitol, le calcipotriène, l'acide calcitroïque, le calcitetrol, la 24-oxo-1alpha,25-dihydroxyvitamine D3, la calcitriol-26,23-lactone, la 1a,25-dihydroxy-cholécalciférol-26,23-peroxylactone, le 26,26,26,27,27,27-hexafluoro-1alpha,25-dihydroxycholécalciférol, la 24-hydroxyvitamine D3, le 1alpha-hydroxy-24-oxocholécalciférol, la calcifédiol lactone, la (23S,25R)-25-hydroxycholécalciférol-26,23-peroxylactone, la 1,25-dihydroxy-24-oxo-vitamine D3, le 24,25-hydroxycholécalciférol, le 25-hydroxy-24-oxocholécalciférol, et le maxalcitol ; et le composé de formule (II) est choisi dans le groupe constitué par l'ergostérol et le 7-déhydrocholesterol.

**4.** Le cocristal de la revendication 3, qui est un cocristal d'un composé de formule (I) choisi dans le groupe constitué par l'ergocalciférol, le cholécalciférol, le calcifédiol, l'ercalcidiol, le calcitriol, l'ercalcitriol et l'alfacalcidol ; et un composé phénolique.

**5.** Le cocristal selon l'une quelconque des revendications 1 à 4, dans lequel le composé phénolique est l'orcinol ou le résorcinol.

**6.** Le cocristal selon la revendication 3, qui est le polymorphe A du cocristal de la vitamine $D_3$ et du résorcinol, **caractérisé en ce qu'**il a un diffractogramme de poudre de rayons X qui comprend des pics caractéristiques à environ 3,6, 11,2, 14,6, 16,2 et 17,0 $\pm$ 0,3 degrés 2 théta (rayonnement de Cu-$K_\alpha$, à $\lambda$ = 1,5418 Å).

**7.** Le cocristal selon la revendication 3, qui est le polymorphe B du cocristal de la vitamine $D_3$ et du résorcinol, **caractérisé en ce qu'**il a un diffractogramme de poudre de rayons X qui comprend des pics caractéristiques à environ 3,5, 9,4, 11,2, 15,7 et 17,5 $\pm$ 0,3 degrés 2 théta (rayonnement de Cu-$K_\alpha$, à $\lambda$ = 1,5418 Å).

**8.** Le cocristal selon la revendication 3, qui est un cocristal de la vitamine $D_2$ et du résorcinol, **caractérisé en ce qu'**il a un diffractogramme de poudre de rayons X qui comprend des pics caractéristiques à environ 3,4, 11,1, 15,5, 16,3 et 17,0 $\pm$ 0,3 degrés 2 théta (rayonnement de Cu-$K_\alpha$, à $\lambda$ = 1,5418 Å).

**9.** Le cocristal selon la revendication 3, qui est un cocristal de la vitamine $D_3$ et de l'orcinol, **caractérisé en ce qu'**il a un diffractogramme de poudre de rayons X qui comprend des pics caractéristiques à environ 3,3, 9,9, 11,4, 16,0 et 17,7 $\pm$ 0,3 degrés 2 théta (rayonnement de Cu-$K_\alpha$, à $\lambda$ = 1,5418 Å).

**10.** Le cocristal selon la revendication 3, qui est le polymorphe A du cocristal de la vitamine $D_2$ et de l'orcinol A, **caractérisé en ce qu'**il a un diffractogramme de poudre de rayons X qui comprend des pics caractéristiques à environ 3,2, 9,8, 11,2, 15,7 et 17,5 $\pm$ 0,3 degrés 2 théta (rayonnement de Cu-$K_\alpha$, à $\lambda$ = 1,5418 Å).

**11.** Le cocristal selon la revendication 3, qui est le polymorphe B du cocristal de la vitamine $D_2$ et de l'orcinol, **caractérisé en ce qu'**il a un diffractogramme de poudre de rayons X qui comprend des pics caractéristiques à environ 3,1, 9,7, 14,6, 17,0 et 17,3 $\pm$ 0,3 degrés 2 théta (rayonnement de Cu-$K_\alpha$, à $\lambda$ = 1,5418 Å).

**12.** Une composition comprenant une quantité efficace du cocristal d'un composé de formule (I) ou de formule (II) et d'un coformateur donneur de liaison hydrogène tel que défini dans l'une quelconque des revendications 1 à 11 avec un ou plusieurs excipients ou véhicules acceptables appropriés.

**13.** La composition selon la revendication 12, qui est une composition pharmaceutique ou vétérinaire, une composition cosmétique, un supplément nutritionnel comprenant un aliment fonctionnel et des boissons fonctionnelles, un aliment médical, un prémélange, un aliment pour des animaux ou un aliment pour des animaux de compagnie.

**14.** Un produit de soins personnels comprenant la composition telle que définie dans la revendication 12.

**15.** Un cocristal d'un composé de formule (I) ou de formule (II) et d'un coformateur donneur de liaison hydrogène tel que défini dans l'une quelconque des revendications 1 à 12 pour son utilisation en tant que médicament.

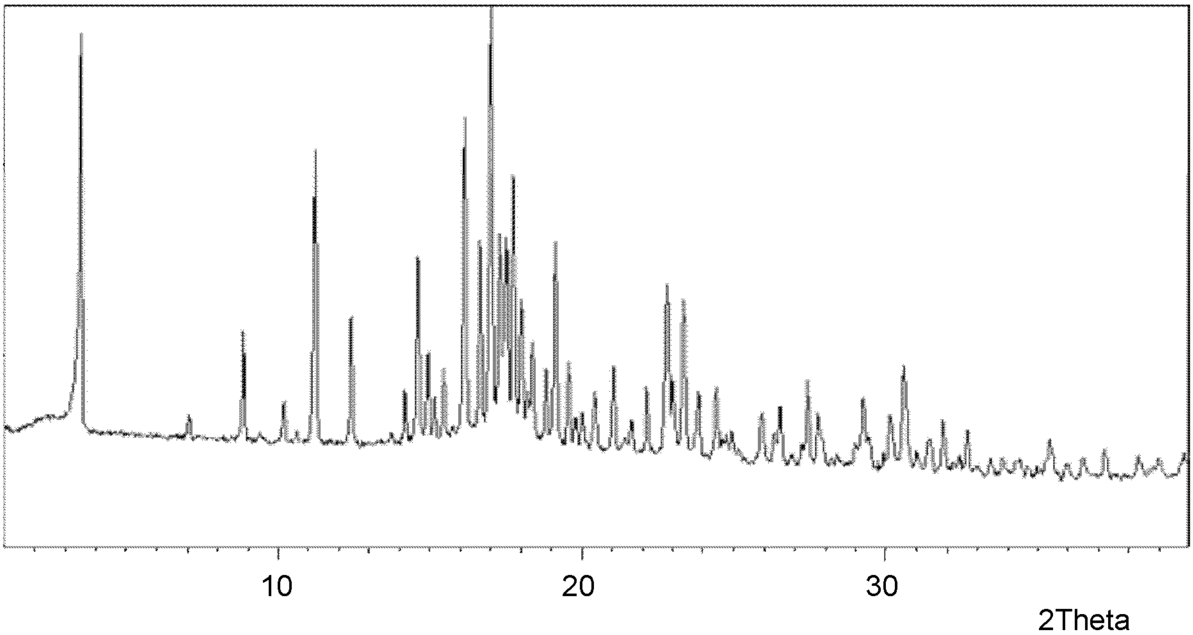


**Fig.1**

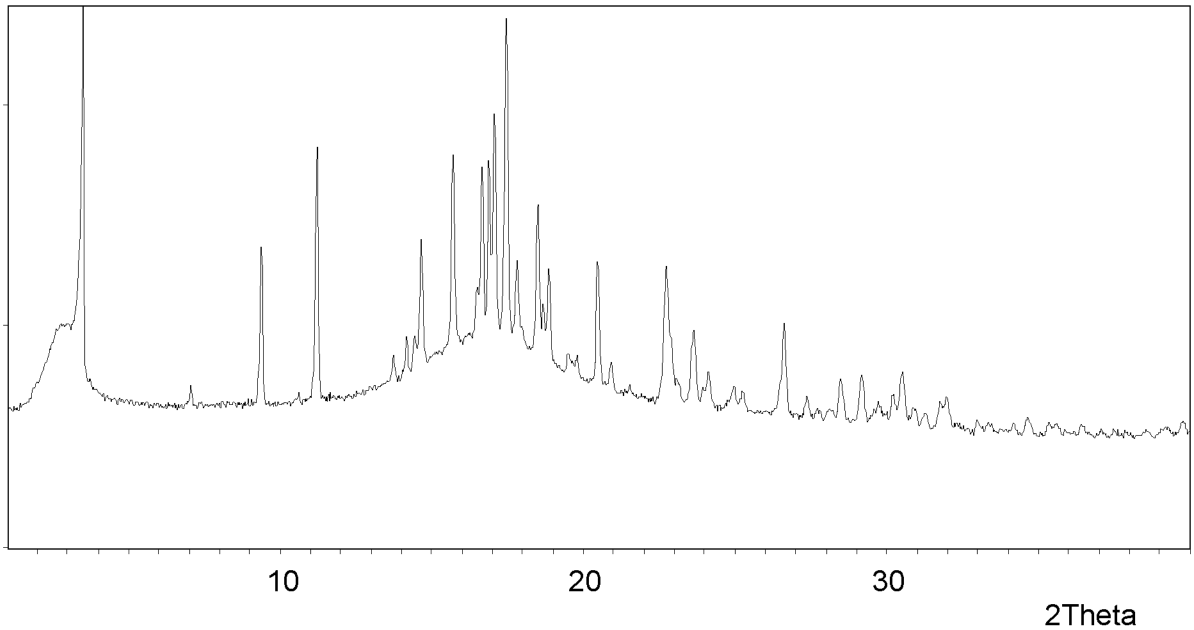


**Fig. 2**

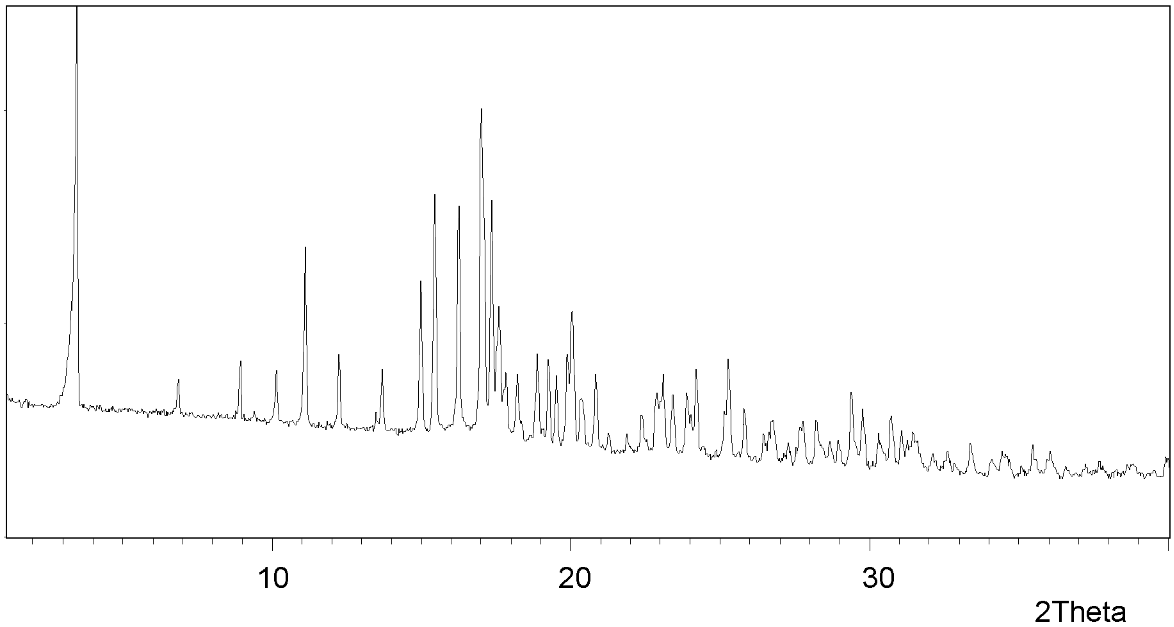


Fig. 3

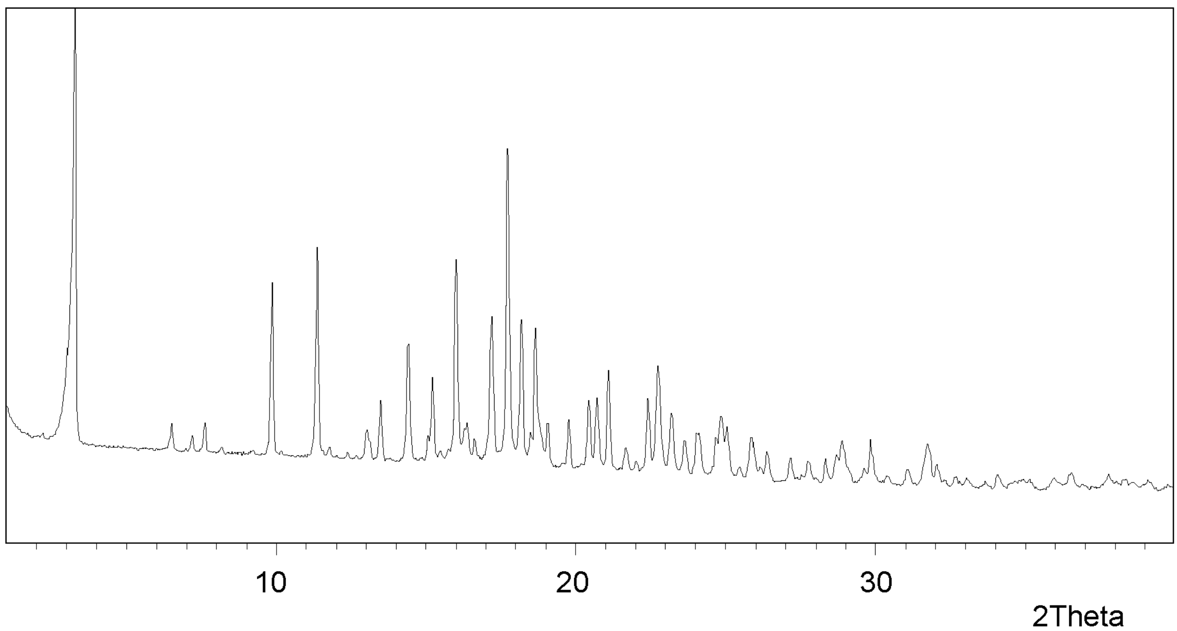


Fig. 4

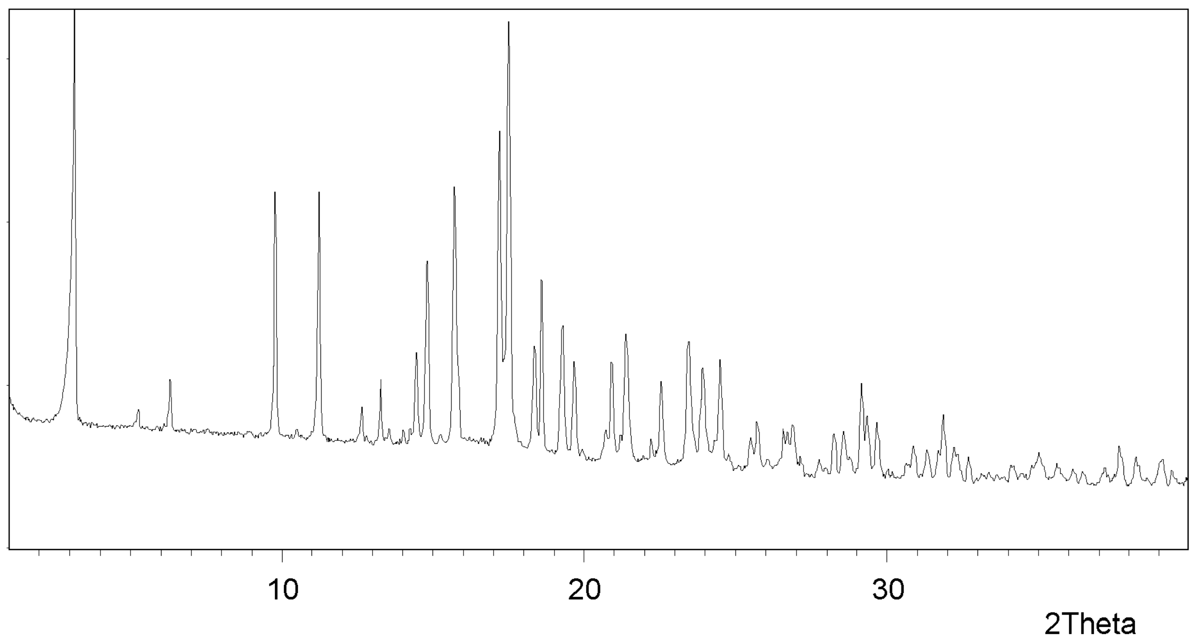


Fig. 5

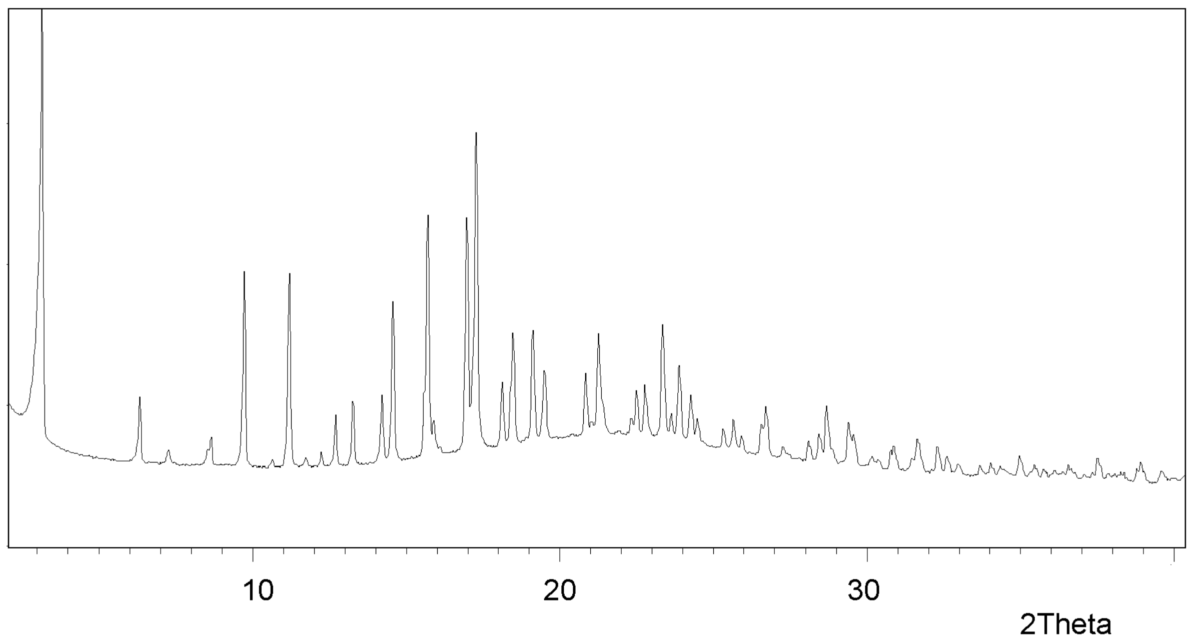


Fig. 6

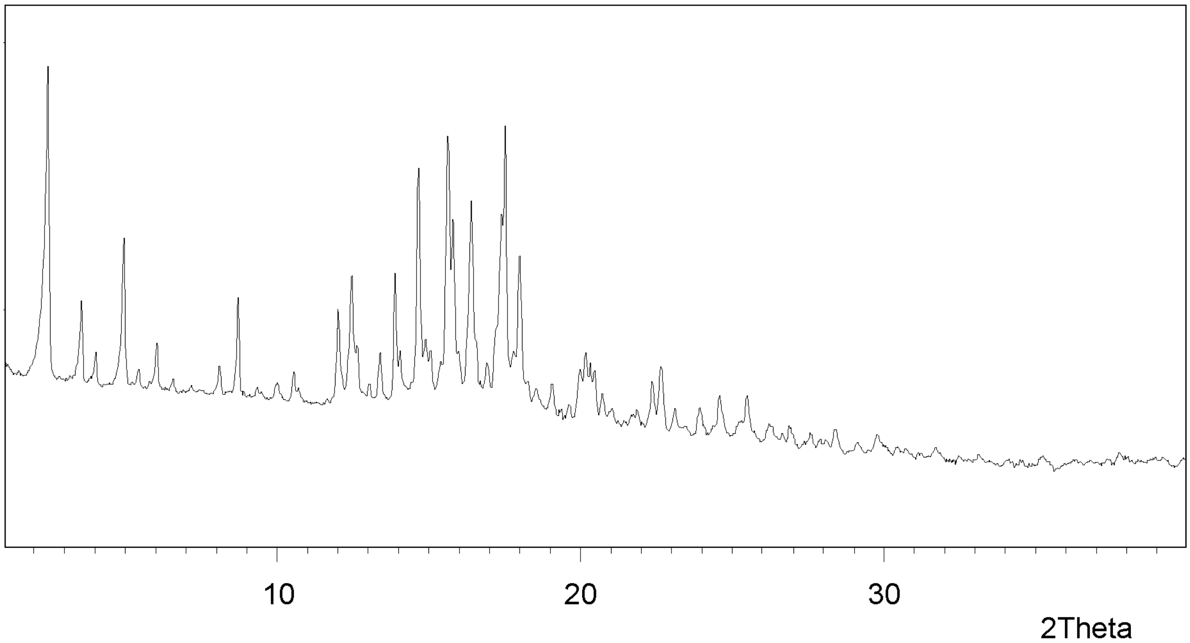


**Fig. 7**

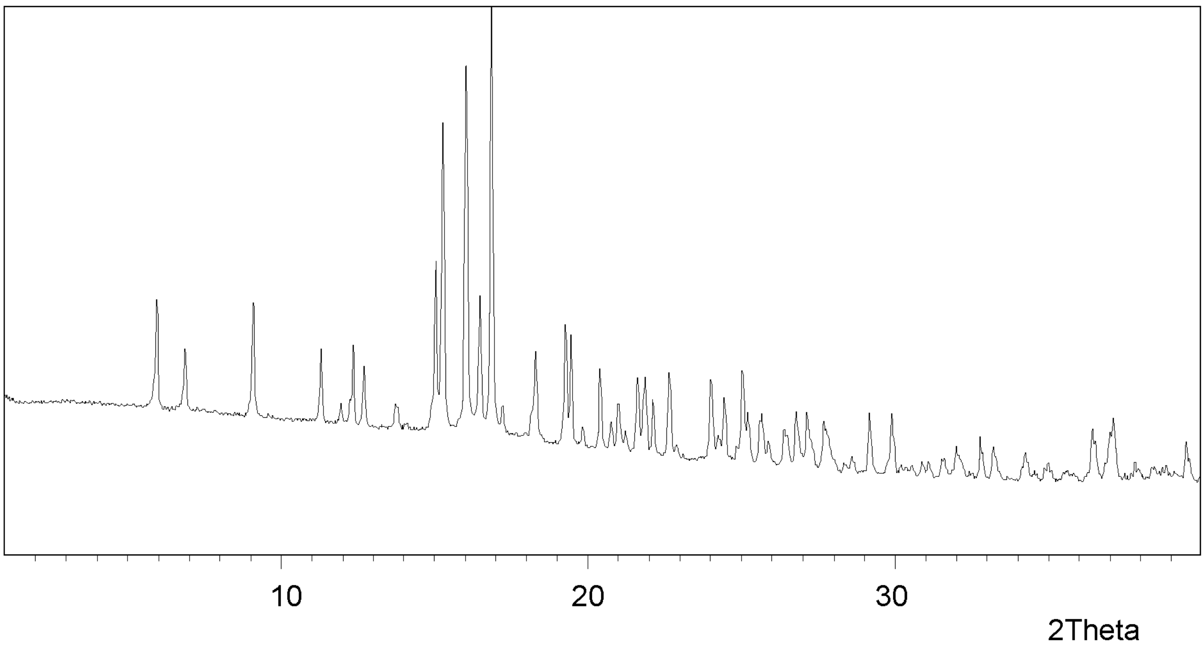


**Fig. 8**

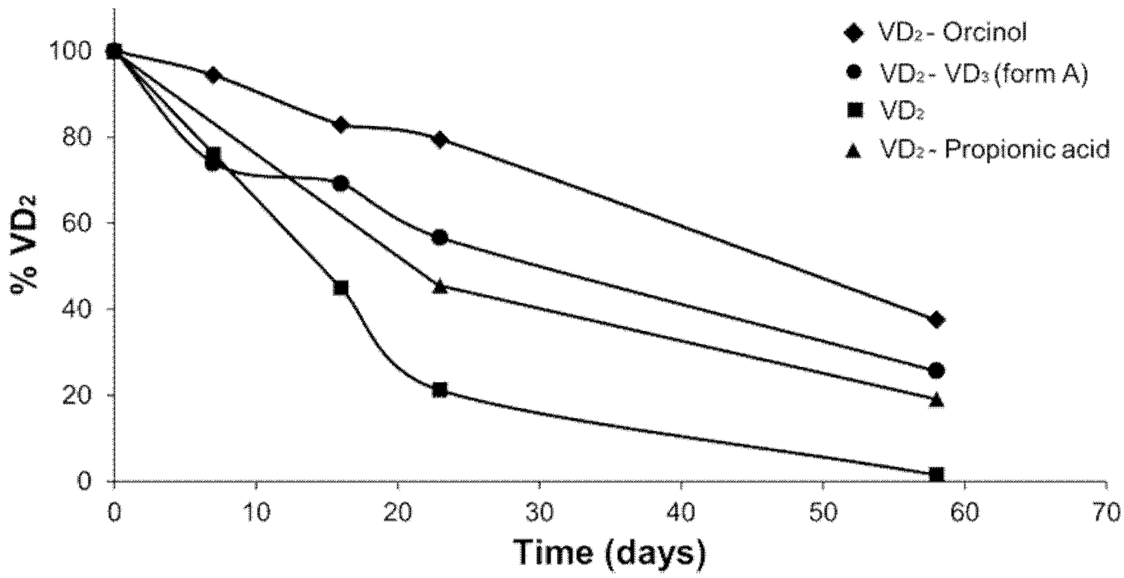
VD2 - Orcinol
VD2 - VD3 (form A)
VD2
VD2 - Propionic acid
% VD2
Time (days)
0
20
40
60
80
100
0
10
20
30
40
50
60
70

Fig. 9

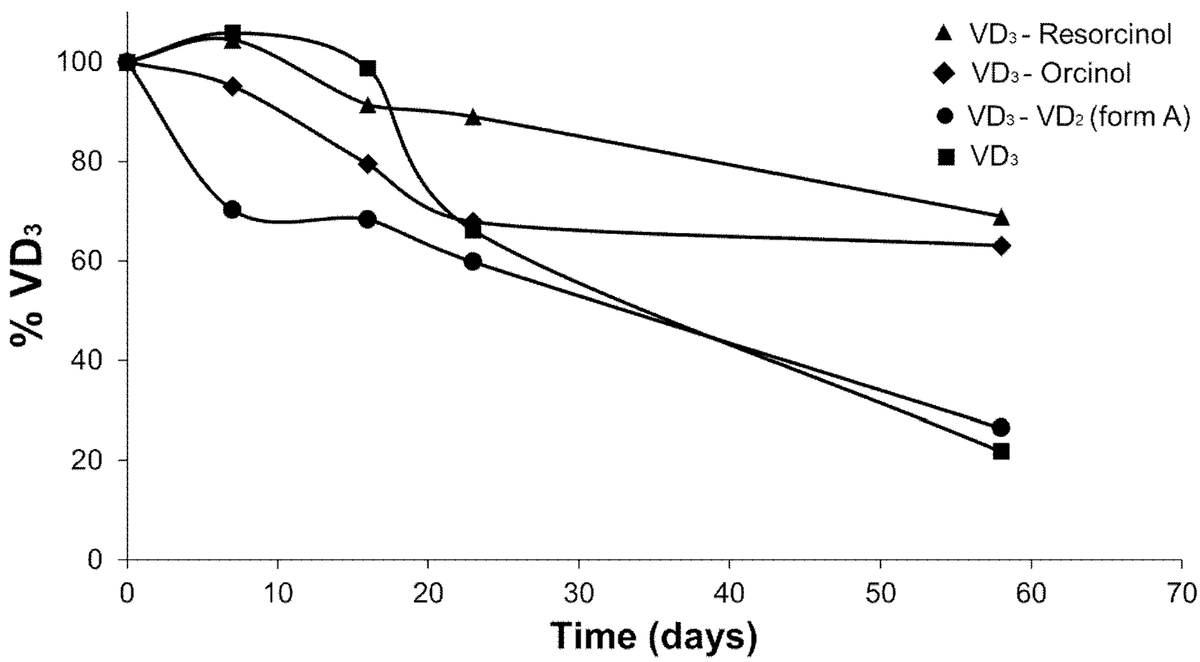
VD3 - Resorcinol
VD3 - Orcinol
VD3 - VD2 (form A)
VD3
% VD3
Time (days)
0
20
40
60
80
100
0
10
20
30
40
50
60
70

Fig. 10

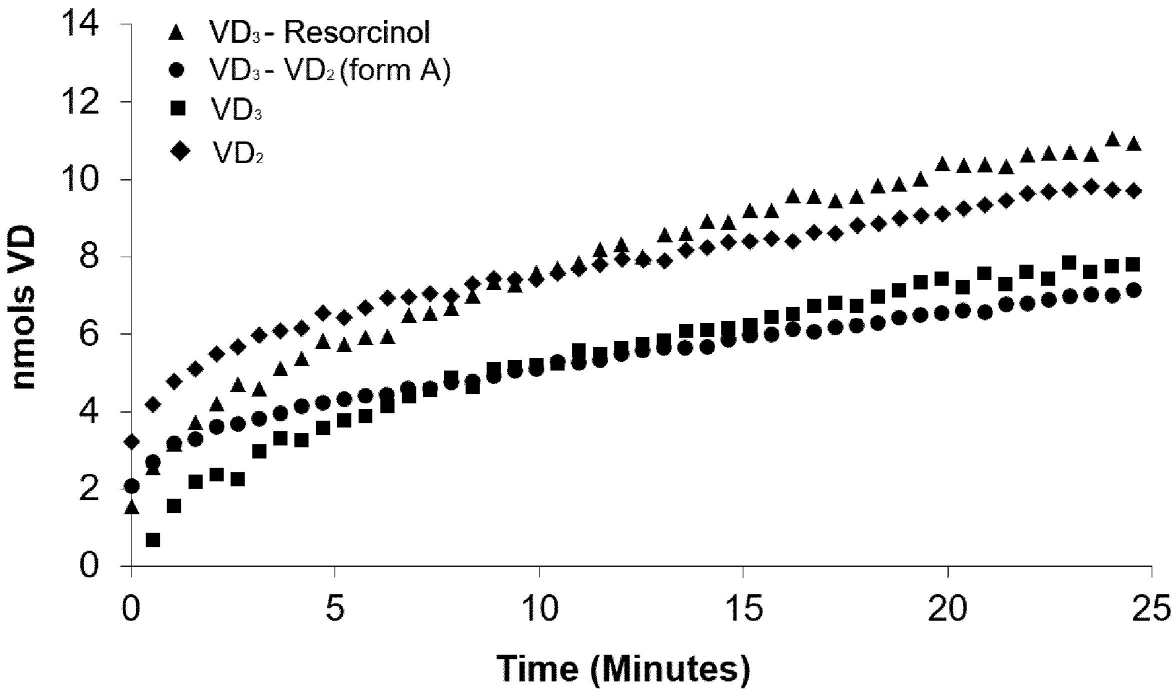
▲ VD3 - Resorcinol
● VD3 - VD2 (form A)
■ VD3
◆ VD2
nmols VD
Time (Minutes)
0
2
4
6
8
10
12
14
0
5
10
15
20
25

Fig. 11

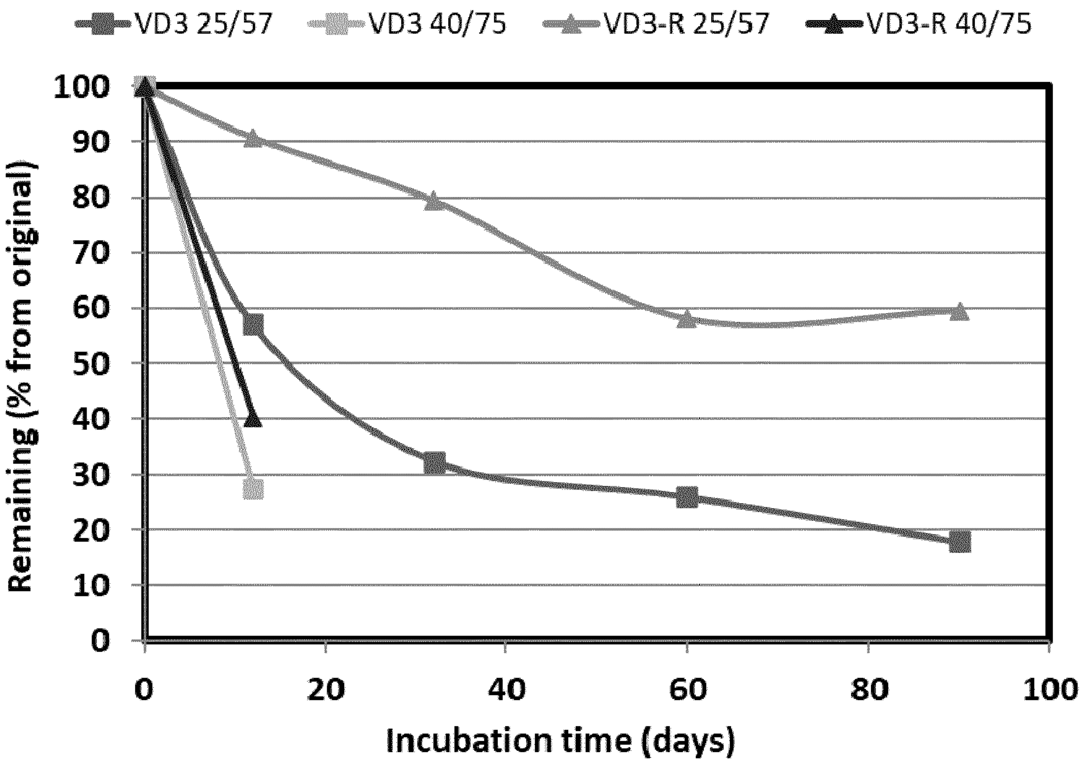
VD3 25/57
VD3 40/75
VD3-R 25/57
VD3-R 40/75
Remaining (% from original)
Incubation time (days)
0
10
20
30
40
50
60
70
80
90
100
0
20
40
60
80
100

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 19383040
- WO 2008153945 A
- WO 2019154237 A


### Non-patent literature cited in the description


- **WANG et al.** *Chem. Commun.,* 2016, vol. 52, 3572
- **T. GRAVESTOCK et al.** *Anal. Methods,* 2011, vol. 3, 560
- **WANG, JR et al.** Stabilizing vitamin D by conformationally selective co-crystallization. *Chem. Commun.,* 2013, vol. 50, 855-858
- **WANG J.R. et al.** Drug-drug co-crystallization presents a new opportunity for the development of stable vitamins. *Chem. Commun.,* 2016, vol. 52, 3572-3575